# EUROPEAN PATENT SPECIFICATION

(11) **EP 4 142 508 B1**
(45) Date of publication and mention of the grant of the patent: **09.10.2024**
(21) Application number: 21726769.9
(22) Date of filing: 29.04.2021
(51) Int. Cl.: A23L 7/10, A61K 8/21, A61K 8/49, A23L 27/20, A61Q 11/00, A23L 27/10, A23G 4/06

(54) **MINT FLAVOR COMPOSITIONS**
MINZGESCHMACKSZUSAMMENSETZUNGEN
COMPOSITIONS D'ARÔME DE MENTHE

(30) Priority: 01.05.2020 US 202063018525 P
(43) Date of publication of application: 08.03.2023
(73) Proprietor: The Procter & Gamble Company, Cincinnati, OH 45202 (US)
(72) Inventor: MORGAN, George, Kavin, III, Cincinnati, Ohio 45202 (US); SANKER, Lowell, Alan, Cincinnati, Ohio 45202 (US); ANDERSON, Dawn, Louise, Cincinnati, Ohio 45202 (US); HOKE, Steven, Hamilton, II, Cincinnati, Ohio 45202 (US); LEI, Qingxin, Cincinnati, Ohio 45202 (US)
(74) Representative: P&G Patent Germany
(86) International application number: PCT/US2021/029773
(87) International publication number: WO 2021/222486

(56) References cited:
- WO-A1-2019/215074
- JP-B1- 6 530 853
- US-A1- 2019 022 264
- US-A1- 2019 022 265
- ASAI ET AL: "Enhancer for fragrance composition for increasing savoriness of mint flavor oral products used for in food-drinks or oral care product field comprises compound selected from e.g., mint lactone, menthofurolactone and dihydromint lactone", WPI / 2017 CLARIVATE ANALYTICS,, vol. 2019, no. 46, 12 June 2019 (2019-06-12), XP002802919

## Description

### FIELD OF THE INVENTION

The field of the invention is directed to mint flavor compositions capable of imparting a mint flavor to consumer products, and consumer products containing the same.

### BACKGROUND OF THE INVENTION

JP 6 530853 B1 discloses a flavor enhancer, a flavor-enhancing flavor composition, and flavor for a mint-flavored oral product (a food or drink or an oral care product that has a mint flavor and expresses a mint flavor in the oral cavity).

ASAI ET AL: "Enhancer for fragrance composition for increasing savoriness of mint flavor oral products used for in food-drinks or oral care product field comprises compound selected from e.g., mint lactone, menthofurolactone and dihydromint lactone",WPI / 2017 CLARIVATE ANALYTICS,, vol. 2019, no. 46, 12 June 2019 (2019-06-12) relates to an enhancer for fragrance composition for increasing savoriness of mint flavor oral products used for in food-drinks or oral care product field comprises compound selected from e.g., mint lactone, menthofurolactone and dihydromint lactone.

Mint flavor, and flavors produced from the *Mentha* genus such as peppermint and spearmint, are important for a broad range of consumer product categories including confectionary, personal care, and oral care. Given the ubiquity of mint flavor, many consumers have developed a keen sense of what is expected in a quality mint flavor experience. Furthermore, some consumers have come to additionally expect a cooling sensation in many mint flavored consumer products. Accordingly, the mint flavor profile is often a decisive factor in a consumer's overall rating of a mint-flavored consumer product.

One source of mint flavoring includes incorporating a natural mint oil that is extracted and/or distilled from the *Mentha* genus. A problem with natural sources of mint flavoring, particularly given the complexity of components therein, includes the presence of one or more components that may negatively interact with other formulation ingredients in the consumer product to produce undesirable consequences such as discoloration, negative and/or unstable flavor profiles, and/or rendering active ingredients less effective. A classic example includes a stannous toothpaste containing a natural mint oil. Some of these formulations exhibit stannous acting as a reducing agent to produce sulfurous off-odors over time. This problem is arguably further exacerbated by having natural mint oils collected from different plant varieties, regions, and even different growing seasons to introduce dynamic variables that may need to be accounted in producing the final mint flavor composition and/or overall consumer product formulation. Consequently, this undesirably increases complexity and unpredictability in consumer product formulation design and manufacturing.

There are attempts to formulate a substantially synthetic mint flavor composition. However, these attempts have been met with at least one of many challenges. Firstly, synthetically replicating a mint flavor profile that meets consumers' expectations is very challenging. Indeed, mint oil is complex from a chemical compositional perspective and biological processes of taste and olfaction are also complex. Moreover, many consumers, given the ubiquity of mint flavor, have developed a rather discerning opinion for what is expected in a quality mint flavor profile. Thus, it is challenging enough to develop a substantially synthetic mint flavor that will meet a consumer's expectations for a quality mint flavor profile. However, for this synthetic approach to be commercially viable, especially for lower margin consumer products, the cost should be at least parity, and preferably less expensive, than classic approaches to mint flavoring containing a high amount of natural mint oil.

In summary, there is a need to provide a predominantly synthetic mint flavor composition, that is cost effective, and is essentially parity to mint flavor profiles that are otherwise provided by classic approaches containing a high amount of natural mint oil.

### SUMMARY OF THE INVENTION

The present invention is based on the surprising discovery of predominantly (e.g., greater than 75 wt%, preferably even greater than 90 wt%, of synthetic ingredients) synthetically derived mint flavor compositions that provide a quality mint flavor profile, impart a cooling sensation, and importantly are cost effective. This discovery is based, at least in part, on observations (from more than 100 different formulation iterations) on the role of stereochemistry (e.g., enantiomers) in helping to provide cost-effective solutions to one or more problems described. Specifically, it is surprisingly discovered that by adding certain synthetic, racemic mint flavor components that include non-natural chiral isomer(s) and ratios, it is possible to produce mint flavor compositions that have a pleasing and refreshing mint flavor profile that notably are cost effective to produce. A key cost driver is the significant use of synthetic, racemic sources of mint flavor components, as they are generally more cost effective relative to natural sources or synthetic, pure enantiomers. However, a simple replacement of natural enantiomers with corresponding racemic, synthetic components is not enough for a successful flavor profile. Rather, it is observed that an optimized balance of racemic and pure enantiomeric components is necessary for a successful flavor profile. Furthermore, decreasing or increasing the amount of certain classic mint flavor components can help in achieving this success. And yet further, the addition of certain non-classical components can also help. To this last point, for example, it is also surprisingly discovered that certain components may act as chemical modifiers helping to influence the overall mint flavor profile to achieve this cost-effective solution. These discoveries are contrary to conventional wisdom, which, and without wishing to be bound by theory, suggests the use of racemic/non-natural enantiomeric components in mint oils is viewed as an adulteration of quality natural mint oils due to strong (unbalanced) or different odor characters and/or weaker cooling profiles exhibited by non-natural enantiomers. By selectively balancing these enantiomers and/or use of certain components and/or adjusting levels of certain components, mint flavor compositions with satisfactory mint flavor profiles can be created from predominantly synthetic ingredients, and consequently, a relatively high use of non-naturally occurring enantiomers.

The present invention provides a mint flavor composition comprising:
(a) a mint flavor component that is dihydromint lactone, wherein the dihydromint lactone is from 0.035 - 0.500, preferably 0.040 - 0.300, more preferably 0.045 - 0.100 of peak area percent, as determined by Lei-Hoke Method I and wherein the composition comprises from about 0.025% to about 0.750% by weight of mint flavor composition of racemic dihydromint lactone, (-)-dihydromint lactone, (+)-dihydromint lactone, and/or combinations thereof; and
(b) an additional mint flavor component, wherein the additional mint flavor component is selected from menthone, isomenthone, alpha-pinene, beta-pinene, limonene, menthol, neomenthol, isomenthol, neoisomenthol, menthyl acetate, linalool, terpinen-4-ol, isopulegol, piperitone, eucalyptol, thymol, viridiflorol, 3-hexen-1-ol, menthofuran, caryophyllene, carvone, sabinene, myrcene, camphene, alpha-terpinene, cis-ocimene, alpha-thujene, delta-3-carene, gamma-terpinene, 3-octanol, trans-sabinene hydrate, germacrene D, delta-cadinene, p-cymene, pulegone, and alpha-terpineol, or combinations thereof.

An aspect of the invention provides a mint flavor composition according to the appended claims comprising: from 9.2 - 20, preferably 9.5 - 15, more preferably 10.0 - 13, of peak area percent of mint components that are C₁₀H₁₆ monoterpenes, as determined by Lei-Hoke Method I, and an additional mint flavor component according to claim 1.

Another aspect of the invention provides a mint flavor composition according to the appended claims comprising: a mint flavor component that is alpha-pinene, wherein the alpha-pinene has: (a) a peak area percent from 1.90 - 5, preferably 2.00 - 4, more preferably 2.20 - 3.5, as determined by Lei-Hoke Method I; and (b) a peak area ratio of (-)-alpha-pinene : (+)-alpha-pinene from 3.0 - 6, preferably 3.1 - 5, more preferably 3.2 - 4.7, as determined by Lei-Hoke Method IV; and an additional mint flavor component according to claim 1.

Another aspect of the invention provides a mint flavor composition according to the appended claims comprising: a mint flavor component that is from 1.1 - 5, preferably 1.2 - 3, more preferably 1.5 - 2.5, of peak area percent of (-)-beta-pinene, as determined by Lei-Hoke Method V; and an additional mint flavor component according to claim 1.

Another aspect of the invention provides a mint flavor composition according to the appended claims comprising: a mint flavor component that is from 0.117 - 0.2, preferably 0.120 - 0.200, more preferably 0.125 - 0.190 of peak area percent of (-)-linalool, as determined by Lei-Hoke Method V; and an additional mint flavor component according to claim 1.

Another aspect of the invention provides a mint flavor composition according to the appended claims comprising: a mint flavor component that is (+)- and (-)- menthol; wherein the (+)- and (-)- menthol has a peak area percent of 40.0 - 45.0, preferably 41.5 - 45.0, as determined by Lei-Hoke Method I; wherein a peak area ratio of (+)-menthol : (-)-menthol is from 0.2 - 0.4, preferably 0.21 - 0.35, as determined by Lei-Hoke Method II; and an additional mint flavor component according to claim 1.

Another aspect of the invention provides a mint flavor composition according to the appended claims comprising: a mint
flavor component that is dihydromint lactone, wherein the dihydromint lactone is from 0.035 - 0.500, preferably 0.040 - 0.300, more preferably 0.045 - 0.100 of peak area percent, as determined by Lei-Hoke Method I; and an additional mint flavor component according to claim 1.

Another aspect of the invention provides a mint flavor composition comprising: a mint flavor component that is menthyl acetate, wherein the menthyl acetate has a peak area percent from 5.5 - 6.5, preferably 5.8 - 6.5, as determined by Lei-Hoke Method I; wherein a peak area ratio of (+)-menthyl acetate : (-)-menthyl acetate is from 0.1 - 0.980, preferably 0.7 - 0.980, as determined by Lei-Hoke Method II; and an additional mint flavor component.

Another aspect of the invention provides a mint flavor composition comprising: a mint flavor component that is (+)- and (-)-menthone, wherein the (+)- and (-)-menthone has a peak area percent of 21 - 26, preferably 22.0 - 26.0, as determined by Lei-Hoke Method I; wherein a peak area ratio of the (+)-menthone : (-)-menthone is from 0.9 - 1, preferably 0.91 - 0.99, as determined by Lei-Hoke Method III; and an additional mint flavor component.

Another aspect of the invention provides for a mint flavor composition according to the appended claims comprising greater than 80 weight percent (wt%), preferably greater than 85 wt%, more preferably greater than 90 wt%, even more preferably 93 wt% of synthetic components.

Another aspect not part of the invention provides for a method of making a flavor / mint flavor composition comprising the steps: (a) steam distilling *Mentha* genus plant matter to produce a first mint distillate, wherein the first mint distillate comprises at least 25 peak area percent of limonene, as determined by Lei-Hoke Method I; wherein the first mint distillate further comprises at least 25 peak area percent of one or more mint flavor components, as determined by Lei-Hoke Method I, wherein each of these mint flavor components have a boiling point from 155 - 183 degrees Celsius; and (b) combining the produced first mint distillate to an additional mint flavor component according to claim 1 such that the first mint distillate comprises 0.5% - 6.0% by weight of the flavor / mint flavor composition.

Another aspect not part of the invention provides for a method of making a consumer product
comprising the step of combining an aforementioned flavor and a carrier to make the consumer product.

Another aspect of the invention provides a consumer product comprising an aforementioned mint flavor composition or an aforementioned flavor and an optional carrier.

An advantage provided in the use of predominantly synthetic ingredients in the mint flavor compositions herein is the reduction of seasonal or geographical variations in natural mint composition, quality, sensory, character, and/or cost that otherwise may be exhibited by natural mint oils.

An advantage provided in the use of predominantly synthetic ingredients in the mint flavor compositions herein is to help provide sensory stability (e.g., flavor profiles) in consumer formulations, especially those containing reducing agents such as stannous ions.

An advantage provided in the use of predominantly synthetic ingredients, while minimizing ingredients that otherwise do not materially contribute to the flavor profile, generally helps to minimize negative interactions with other formulation ingredients (e.g., stannous ions).

An advantage provided in the use of certain synthetic, racemic sources of mint flavor components is cost savings.

An advantage in the mint flavor compositions herein (and flavor and consumer products containing the same) are consumer favorable mint flavor profiles. The flavors have favorable aroma profiles and also display well once in the context of the finished consumer product.

These and other features, aspects, and advantages of the present invention will become evident to those skilled in the art from the detailed description which follows.

### BRIEF DESCRIPTION OF THE DRAWINGS

While the specification concludes with claims particularly defining and distinctly claiming the invention, it is believed that the invention will be better understood from the following description of the accompanying figures:
Figure 1 is a table comparison of peak area percent and enantiomeric peak area ratio of certain mint flavor components in inventive and comparative examples;
Figure 2 is a table comparison of peak area percent and enantiomeric peak area ratio of certain additional mint flavor components in inventive and comparative examples;
Figure 3 is a table comparison of peak area percent and enantiomeric peak area ratio of certain further additional mint flavor components in inventive and comparative examples;
Figure 4 is a table comparison of peak area percent of mint flavor components in inventive and comparative examples;
Figure 5 is a table comparison of peak area percent of additional mint flavor components in inventive and comparative examples;
Figure 6 is a table comparison of peak area percent of further additional mint flavor components in inventive and comparative examples;
Figure 7 is a table comparison of peak area percent and peak area ratio of certain mint flavor component terpenes in inventive and comparative examples;
Figure 8 is a table of peak area percent and peak area ratio of menthol in inventive and comparative examples;
Figure 9 is a table of peak area percent and peak area ratio of mint flavor components in inventive and comparative examples;
Figure 10 is a table of peak area percent and peak area ratio of additional mint flavor components in inventive and comparative examples;
Figure 11 is a table of mint flavor components, and their peak area percent, that comprise a first mint distillate (useful in methods of making mint flavor compositions / flavors described herein).

### DETAILED DESCRIPTION OF THE INVENTION

The present invention is defined by the appended claims.

The invention discloses a mint flavor composition comprising:
(a) a mint flavor component that is dihydromint lactone, wherein the dihydromint lactone is from 0.035 - 0.500, preferably 0.040 - 0.300, more preferably 0.045 - 0.100 of peak area percent, as determined by Lei-Hoke Method I and wherein the composition comprises from about 0.025% to about 0.750% by weight of mint flavor composition of racemic dihydromint lactone, (-)-dihydromint lactone, (+)-dihydromint lactone, and/or combinations thereof; and
(b) an additional mint flavor component, wherein the additional mint flavor component is selected from menthone, isomenthone, alpha-pinene, beta-pinene, limonene, menthol, neomenthol, isomenthol, neoisomenthol, menthyl acetate, linalool, terpinen-4-ol, isopulegol, piperitone, eucalyptol, thymol, viridiflorol, 3-hexen-1-ol, menthofuran, caryophyllene, carvone, sabinene, myrcene, camphene, alpha-terpinene, cis-ocimene, alpha-thujene, delta-3-carene, gamma-terpinene, 3-octanol, trans-sabinene hydrate, germacrene D, delta-cadinene, p-cymene, pulegone, and alpha-terpineol, or combinations thereof.

As used herein, the articles including "a", "an", and "the" are understood to mean one or more of what is claimed or described.

As used herein, symbols " / " and " : " are used interchangeably in various contexts to denote a ratio of two items that are placed on either side of the symbol. This ratio, for example, may include a ratio of specific enantiomeric mint flavor components or a peak area ratio of mint flavor components of interest.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of" and "consisting essentially of".

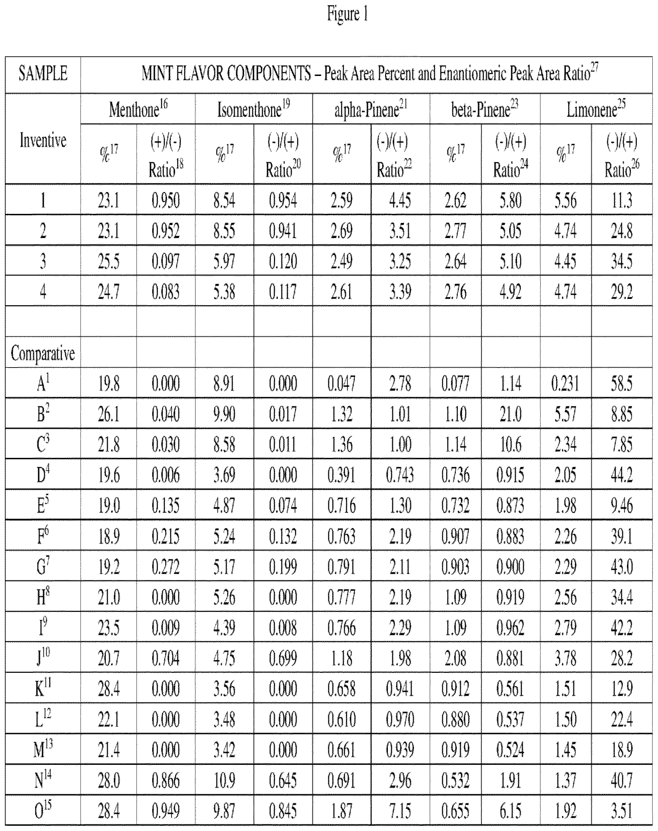

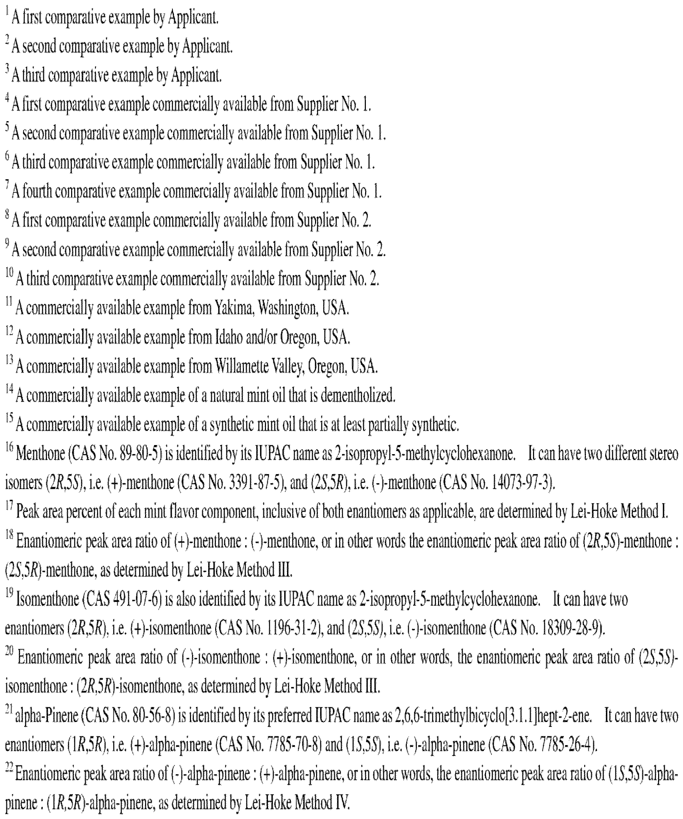

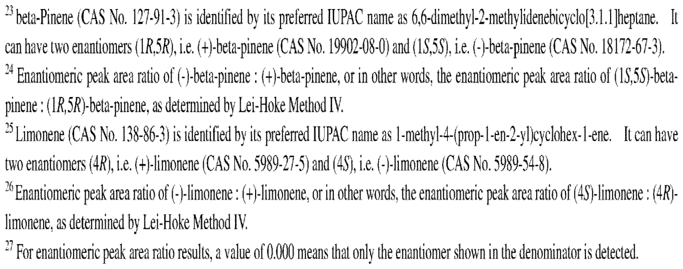

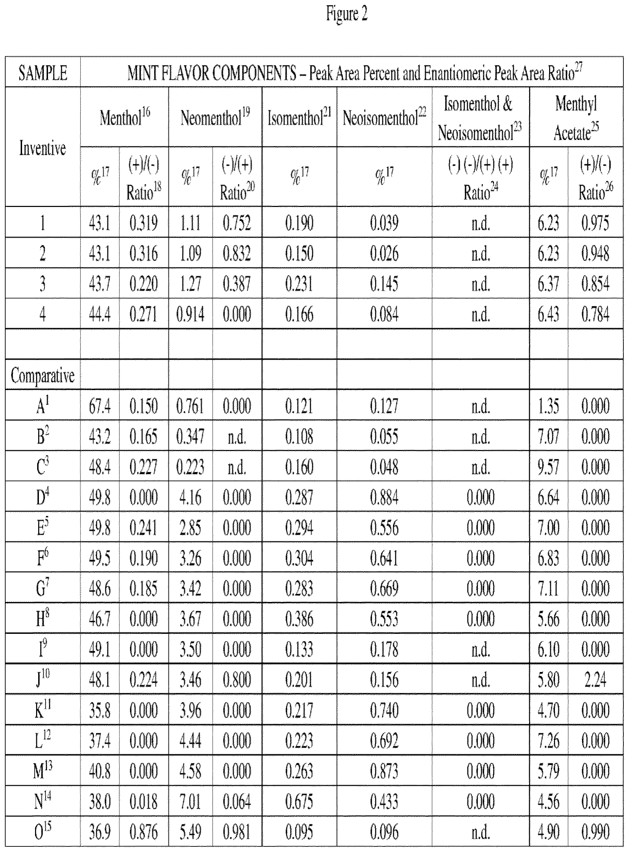

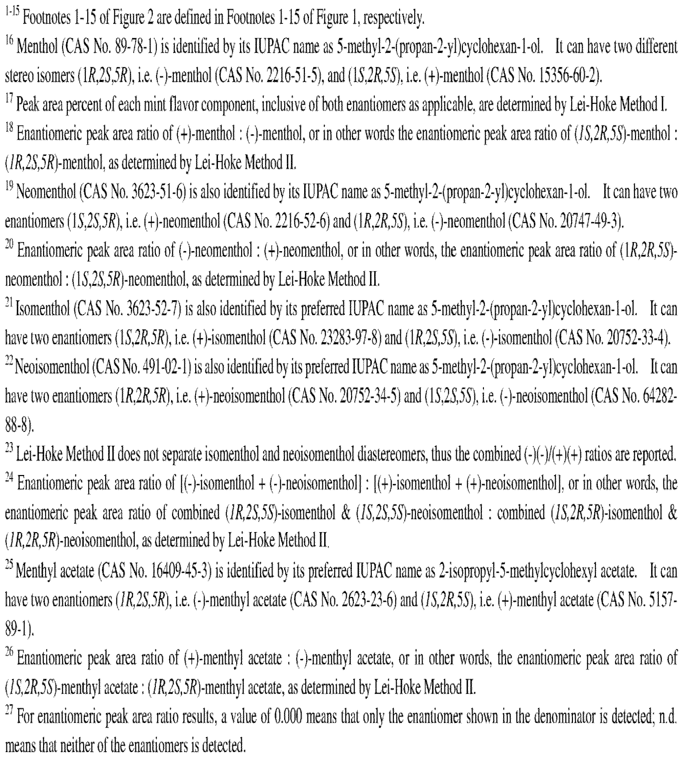

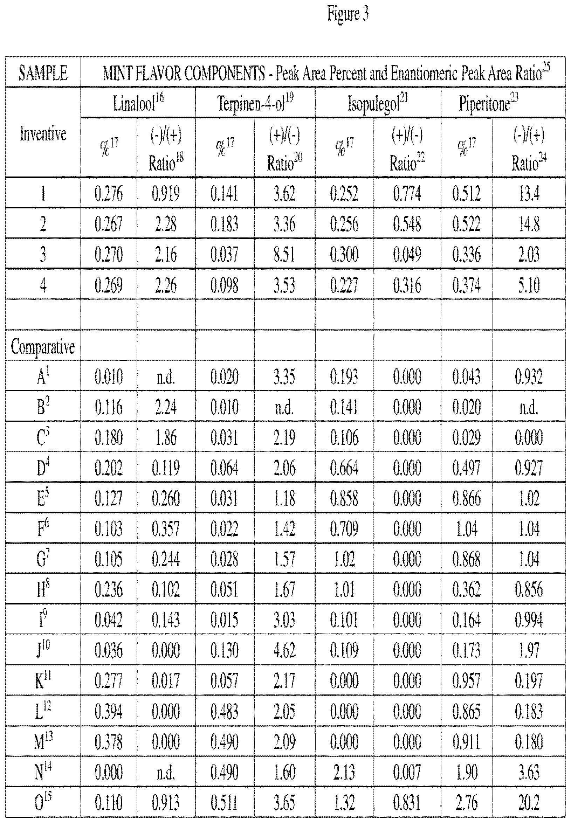

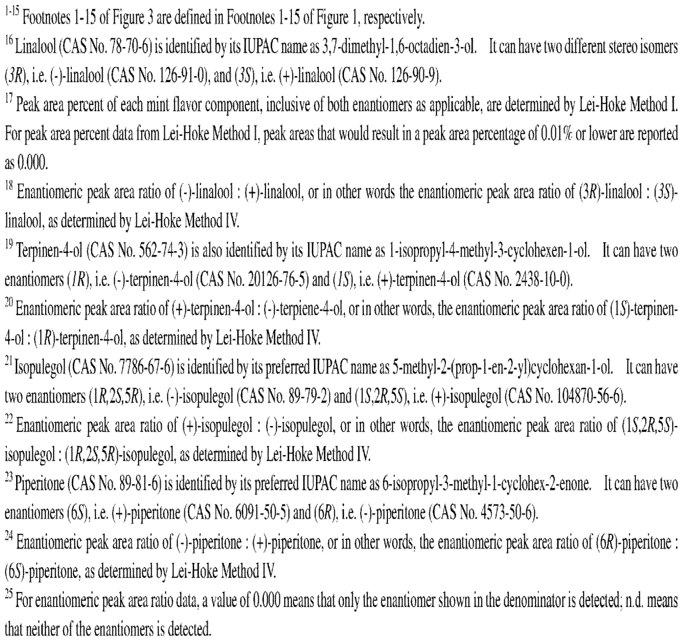

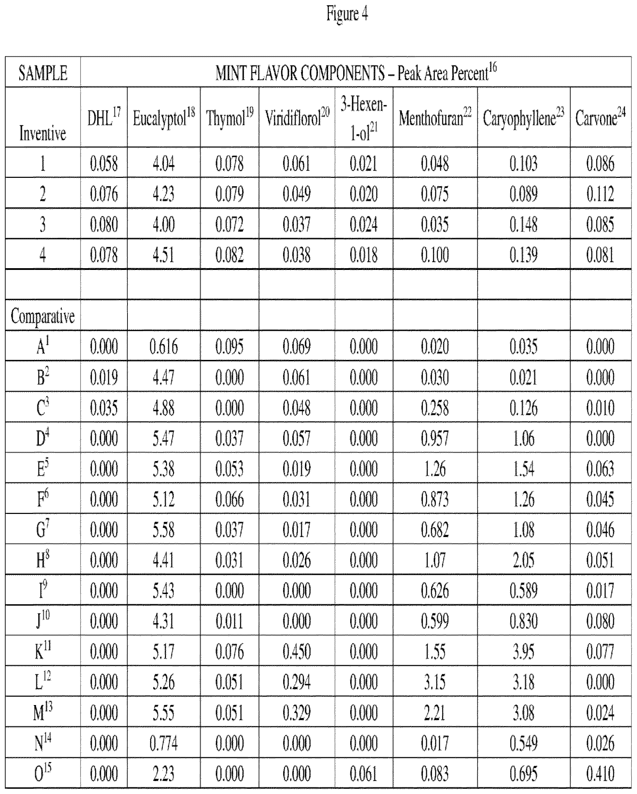

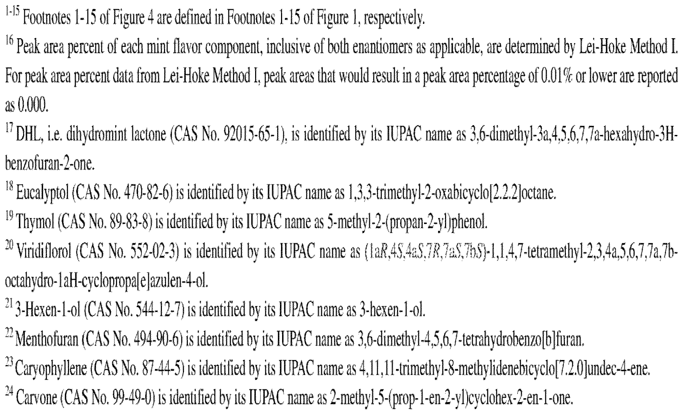

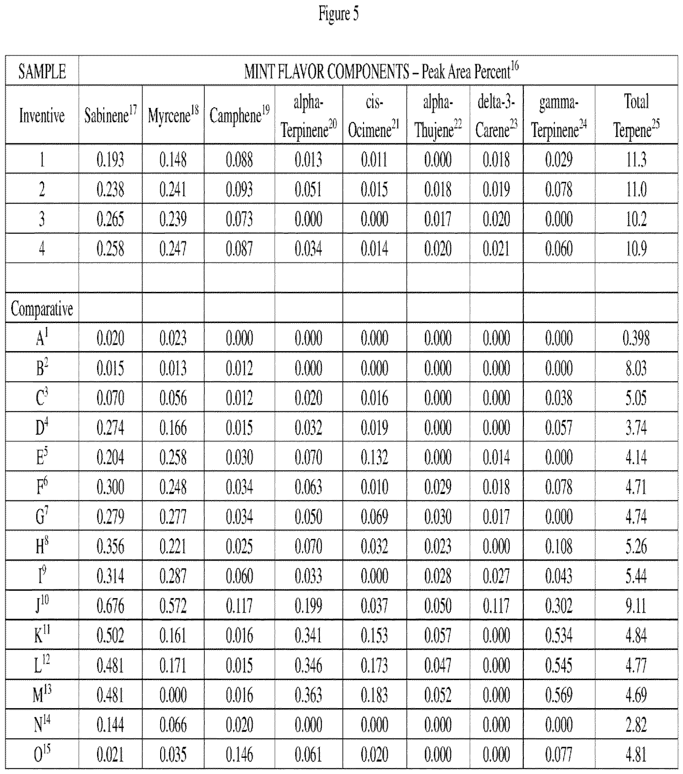

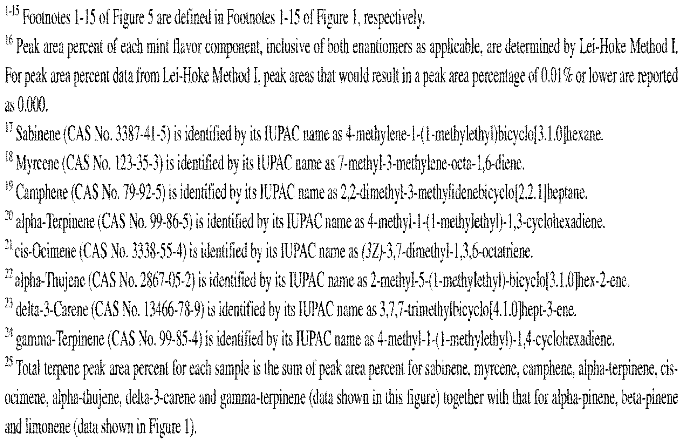

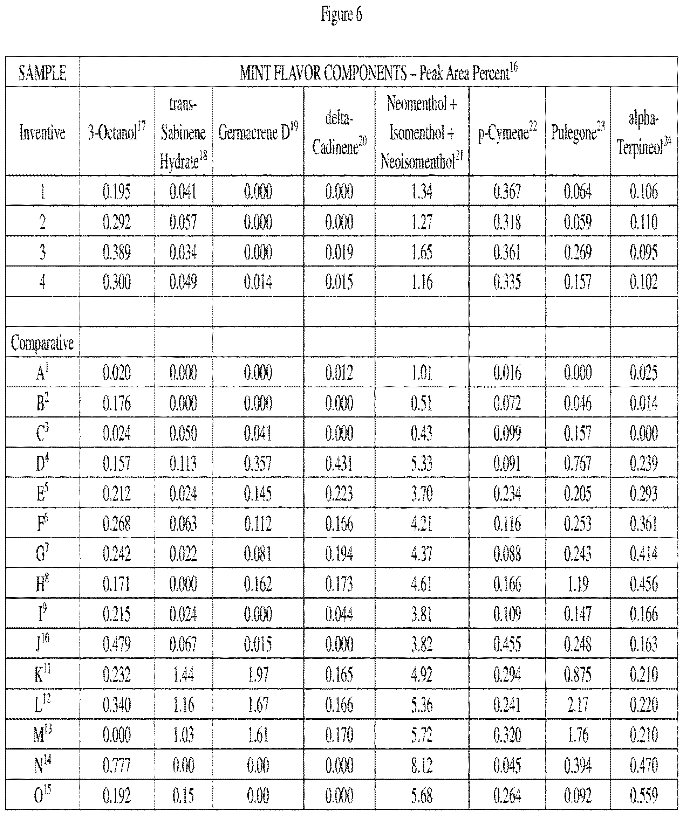

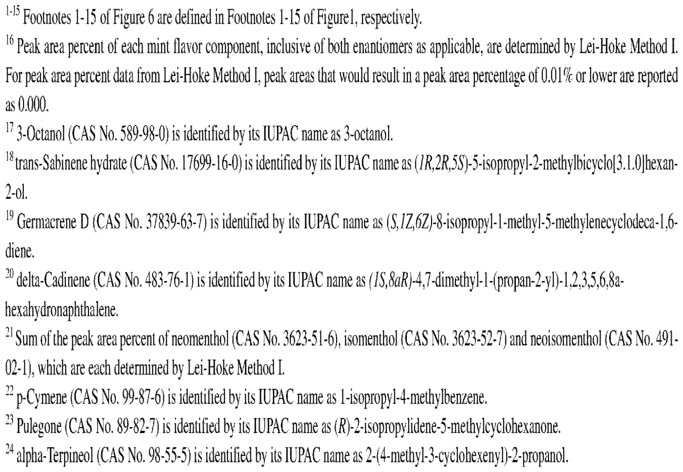

A "mint flavor composition," as used herein, means a composition comprising at least 1 or more, preferably at least 2, more preferably at least 3, yet more preferably at least 4 or more, yet still more preferably from 5-36, alternatively from 10 - 35, 15 - 30, 20 - 31, 25 - 32, or 12 - 29, of the following 37 mint flavor components. In turn, a "mint flavor component" as used herein, means a component selected from the group consisting of menthone, isomenthone, alpha-pinene, beta-pinene, limonene, menthol, neomenthol, isomenthol, neoisomenthol, menthyl acetate, linalool, terpinen-4-ol, isopulegol, piperitone, dihydromint lactone, eucalyptol, thymol, viridiflorol, 3-hexen-1-ol, menthofuran, caryophyllene, carvone, sabinene, myrcene, camphene, alpha-terpinene, cis-ocimene, alpha-thujene, delta-3-carene, gamma-terpinene, 3-octanol, trans-sabinene hydrate, germacrene D, delta-cadinene, p-cymene, pulegone, and alpha-terpineol. In unpublished internal research, flavorists believe a vast majority of these 37 components are generally found in commercially available mint compositions associated with the *Mentha* genus, thus are ostensibly necessary in a composition that provides an acceptable mint profile to users These mint flavor components are identified in the tables of Figures 1-6 and the accompanying footnotes. A mint flavor composition can be contained within a flavor and/or a consumer product.

As used herein, reference to a "mint flavor component" without qualification to its stereochemistry is intended to be inclusive of the component's enantiomers (e.g., racemic mixtures, and the like). In contrast, reference to a specific enantiomer is only intended to include the identified enantiomer. For illustrative purposes, reference to the term "menthol" means the sum of both enantiomers (i.e., (+)- and (-)-menthol), whereas, when referring to a specific enantiomer, it will be identified as such, i.e. either (+)-menthol or (-)-menthol. Specific data on 13 enantiomer pairs is provided in the tables of Figures 1-3.

Generally, flavors are typically chemical components that elicit human perception of aroma, taste, and/or trigeminal impact, and are safe for their intended use in consumer products. Flavors of the present invention comprise a mint flavor composition and optional ingredients. These optional ingredients may include a wide variety of natural and synthetic non-mint flavor components, minors, and/or solvents. One flavor can be combined with a second, third, or more flavors (either directly or sequentially in the making of a consumer product), to provide a final flavor. Without limitation, the flavors of the present invention may be described as having a mint, wintergreen, or spearmint flavor profile. Alternatively, and without limitation, the flavors herein may have one of any of a variety of major flavor profiles such as citrus (e.g., lemon), spice (e.g., cinnamon), or sweet (e.g., vanilla), and wherein the same flavor has mint as a minor note or facet (of the overall flavor profile). The flavors of the present invention may be used within a wide variety of consumer products. That is, flavors of the present invention can be combined with other ingredients (e.g., a carrier) to make a consumer product. The flavor is combined such that it is at a safe and effective level within the consumer product.

A consumer product is the final form which is intended to be used by its end user (i.e., a consumer). Flavors are important for enhancing users' preferences and enjoyment of the consumer product. Non-limiting examples of consumer products include foodstuffs and personal care products. These consumer products can be designed for households or institution users.

### Lei-Hoke Methods I-V

A number of methods are needed for in-depth characterization of mint flavor compositions, especially as many literature methods, such as in J. Rohloff, "Monoterpene Composition of Essential Oil from Peppermint (Mentha x piperita L.) with Regard to Leaf Position Using Solid-Phase Microextraction and Gas Chromatography/Mass Spectrometry Analysis", J. Agric. Food Chem. 47 (1999) 3782 - 3786 and W.M. Coleman, III, B.M. Lawrence, and S.K. Cole, "Semiquantitative Determination of Off-Notes in Mint Oils by Solid-Phase Microextraction", J. Chromatogr. Sci., 40 (2002) 133-139, do not provide for separation of the key enantiomer pairs of major mint flavor components, which is described in M.L. Ruiz del Castillo, G.P. Blanch, and M. Herraiz, "Natural Variability of Enantiomeric Composition of Bioactive Chiral Terpenes in Mentha Piperita", J. Chromatogr. A, 1054 (2004) 87-93 and B.M. Lawrence, "The Composition of Commercially Important Mints", In Mint The genus Mentha, Ed. by Brian M. Lawrence. CRC Press, Taylor & Francis Group, 2007, Chapter 7, pp. 217-324. In this case, where the present mint flavor compositions are defined, at least in part, by selective, optimal usage of non-naturally occurring mint flavor component enantiomers and synthetic racemates, it is necessary to characterize the achiral and chiral mint flavor components of mint flavor compositions. These methods are applicable to flavors and consumer products containing mint flavor compositions. Five methods are provided for this characterization and are summarized in Table A. Due to the challenges of obtaining baseline, or near baseline, separation of the key enantiomeric pairs described, three separate chiral stationary phases are utilized, identified as Lei-Hoke Methods II, III and IV.

**Table A. Methods for Determining Mint Flavor Components in Flavor and Consumer Product Samples.**

| Methods | |
|---|---|
| Name | Description |
| Lei-Hoke Method I; or LHM I | Achiral Determination of Peak Area Percent of Mint Flavor Components in Samples. |
| Lei-Hoke Method II; or LHM II | Chiral Determination of Peak Area Ratios of Menthol, Menthyl Acetate, Neomenthol, and Isomenthol/Neoisomenthol Enantiomer Pairs in Samples. |
| Lei-Hoke Method III; or LHM III | Chiral Determination of Peak Area Ratios of Menthone and Isomenthone Enantiomer Pairs in Samples. |
| Lei-Hoke Method IV; or LHM IV | Chiral Determination of Peak Area Ratios of Seven Enantiomer Pairs of Mint Flavor Components in Samples. |
| Lei-Hoke Method V; or LHM V | Calculation of Peak Area Percent of Individual Mint Flavor Component Enantiomers in Samples. |

### LHM I

Lei-Hoke Method I for Achiral Determination of Peak Area Percent of Mint Flavor Components in Samples is described. Method I contains details for determining peak area percent of mint flavor components in mint flavor compositions contained within flavors and consumer products by Gas Chromatography-Mass Selective Detector ("GC-MSD") with the following sections: (i) sample preparation; (ii) gas chromatographic (GC) separation conditions; (iii) mass spectrometer detector (MSD) calibration; (iv) mass spectrometer data acquisition; and (v) mass spectrometer data processing.

Lei-Hoke Method I: Sample Preparation. Given the numerous components that comprise flavors and the wide range of materials that comprise consumer products, especially given the diversity of product forms including liquids, semi-solids, cremes, gels, lozenges, chewing gums, pastes, solids, etc., a general approach to sample analysis is provided in LHM I. However, given this breadth of possibilities, it is with the expectation, that for any given flavor or consumer product sample, one skilled in the analytical arts conducts sample preparation to assure that the sampling, dilution and/or extraction efficiency confirms representative analysis of greater than 90 weight % (wt%), preferably greater than 95 wt%, of each mint flavor component (relative to the original sample). Specifically, each mint flavor component is made available for analysis by LHM I, regardless of its original matrix, and captured in a form where a representative distribution of mint flavor components is analyzed by LHM I. For example, if using a liquid-liquid extraction, all mint flavor components should be extracted from the consumer product matrix into an organic solvent at greater than 90 wt% and analyzed as detailed in subsequent sections of LHM I described. Confirming greater than 90% extraction efficiency can be accomplished by performing replicate extractions of a given sample followed by analysis of the extracts separately to assure that no significant amounts of mint flavor components are recovered following the initial extraction.

In most cases, mint flavor composition containing samples analyzed by LHM I, whether these samples are from flavors or consumer products, should be extracted or diluted with an organic solvent, such as hexane, prior to injection into a GC-MSD such that the mint flavor composition contained in the sample for injection is approximately 3,000 parts per million (PPM, volume/volume, or v/v) in a liquid. Options for sample preparation to assure representative analysis of greater than 90 wt% of each mint flavor component (relative to the original sample) include: (1) direct analysis of a sample (as applicable); (2) dilution of a sample in an organic solvent or mixture of solvents; or (3) potentially grinding then dispersing and/or mixing of a consumer product sample in an aqueous or aqueous salt solution followed by solid phase or liquid-liquid extraction. When these processes are complete, the mint flavor composition containing sample, or extract thereof, should contain greater than 90 wt% of each mint flavor component, with the total of all mint flavor components at approximately 3,000 PPM (v/v) in a mixture including an organic solvent, such as hexane. This is a target of the mint flavor composition concentration (contained in the sample after preparation for analysis) in a liquid that is suitable for analysis by liquid injection into a GC-MSD according to LHM I.

There are also sample preparation or extraction conditions that should be avoided. For example, static headspace or headspace-solid phase microextraction (HS-SPME) sampling must not be utilized, due to differences in partitioning of mint flavor components. Results from these sample preparations will be different than those obtained by liquid injection and will not accurately represent the mint flavor component peak area percent in the sample. J. Rohloff, J. Agric. Food Chem. 47 (1999) 3782 - 3786; W.M. Coleman et al., J. Chromatogr. Sci., 40 (2002) 133 - 139. Additionally, with static headspace or HS-SPME, it is highly unlikely that greater than 90 wt% of each mint flavor component would be available for sampling and analysis. Likewise, immersion SPME should not be utilized either, as the partitioning of mint flavor components onto the fiber would be selective based on the properties of each mint flavor component and will not accurately represent the peak area percent of each mint flavor component.

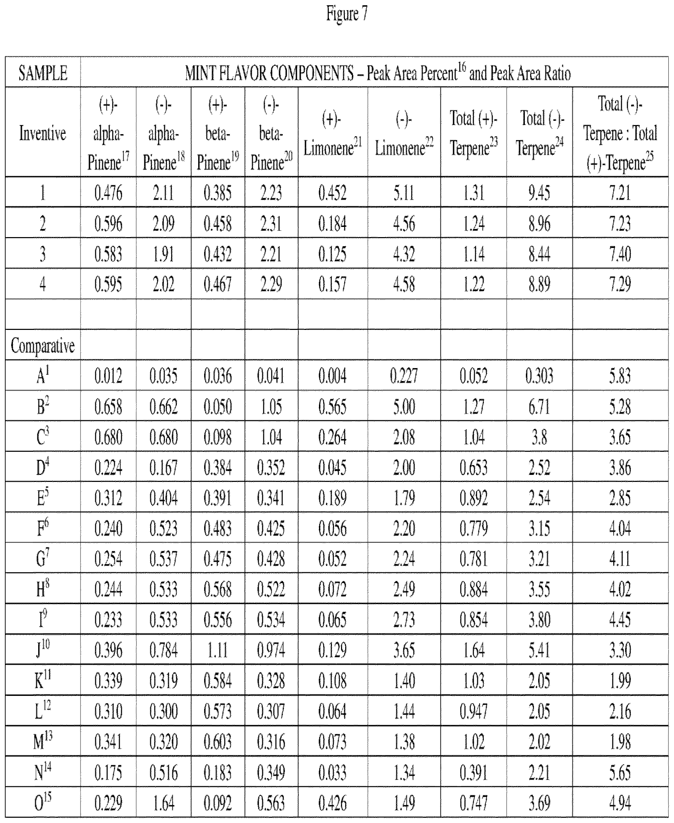

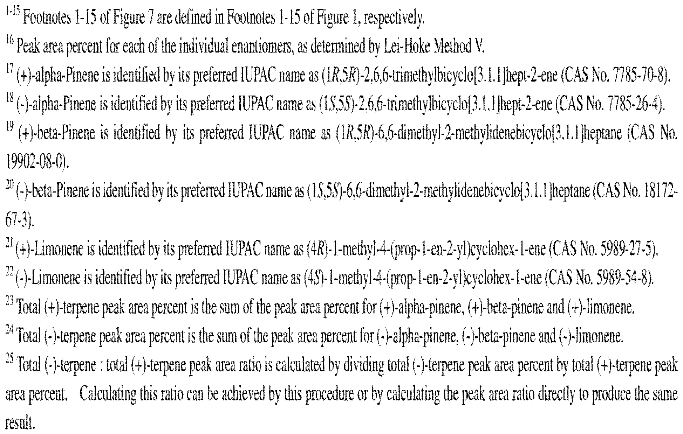

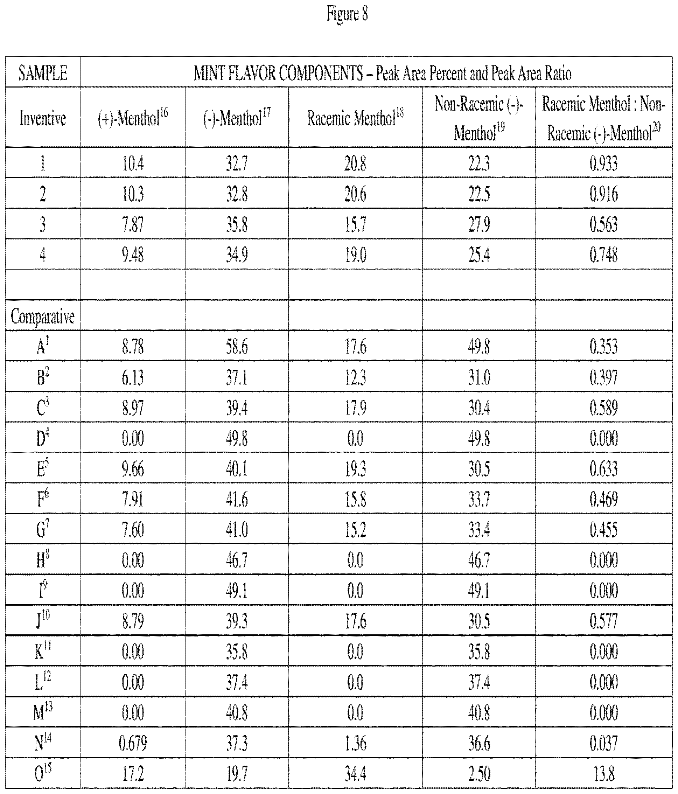

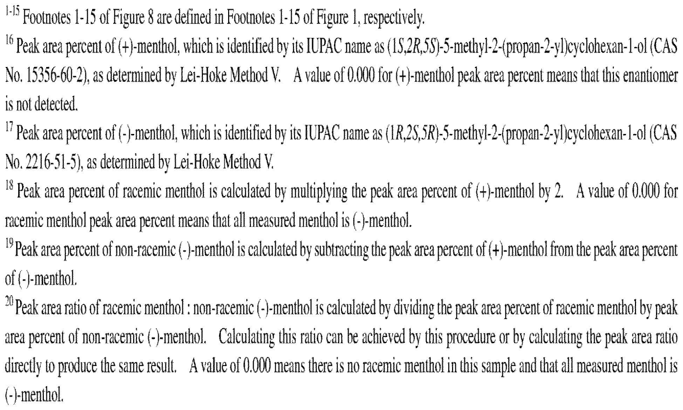

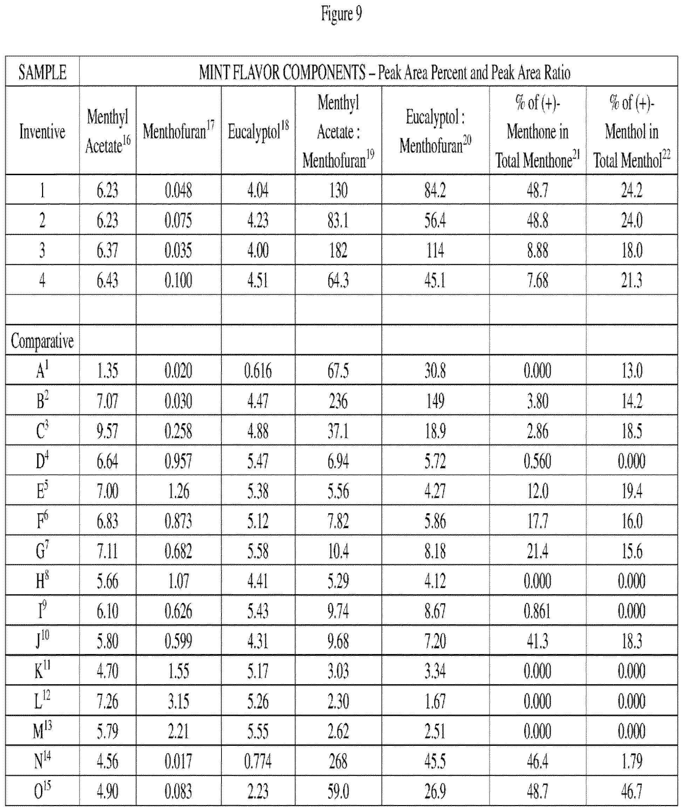

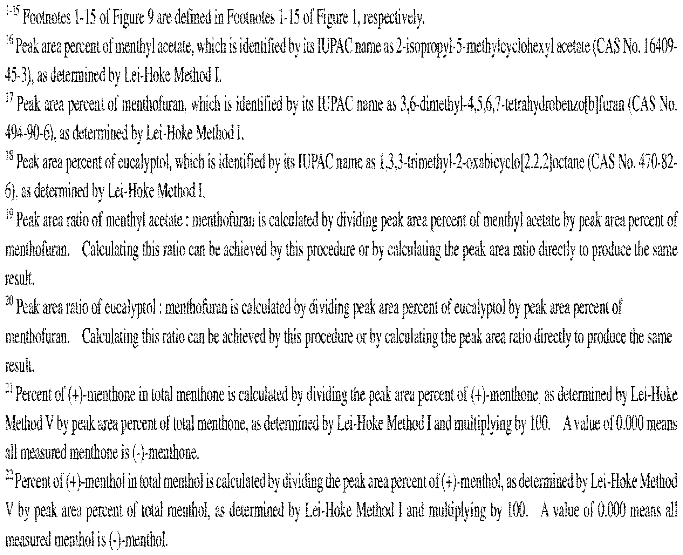

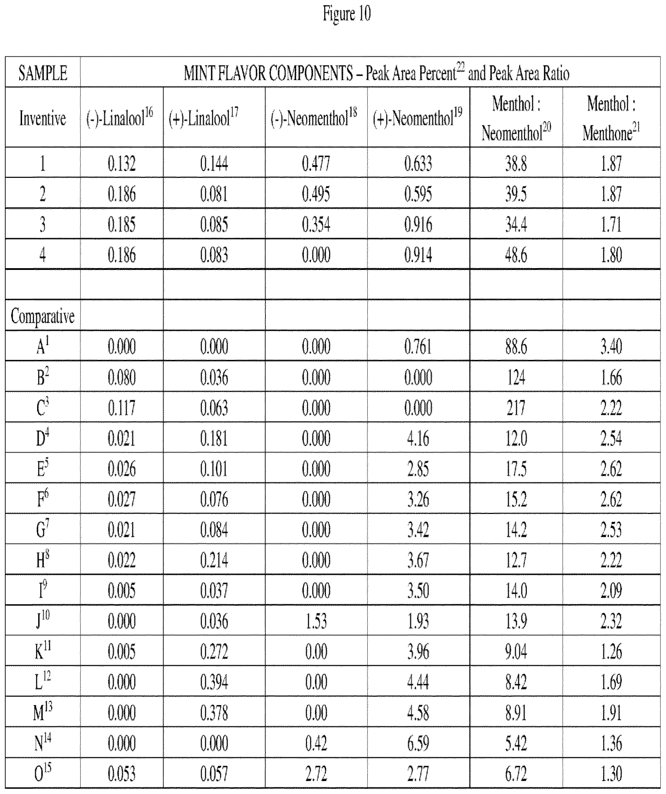

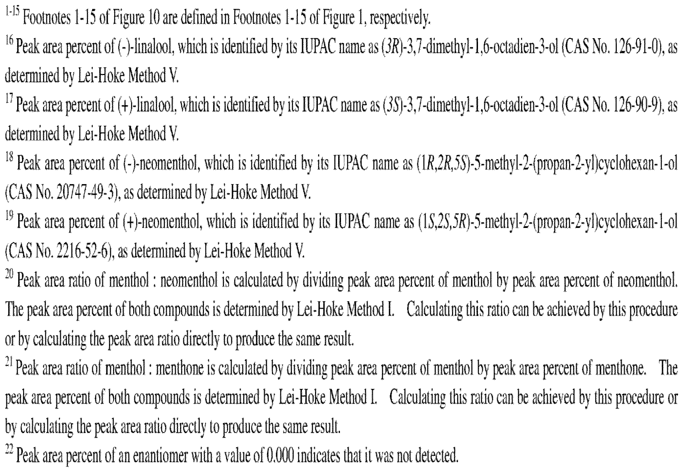

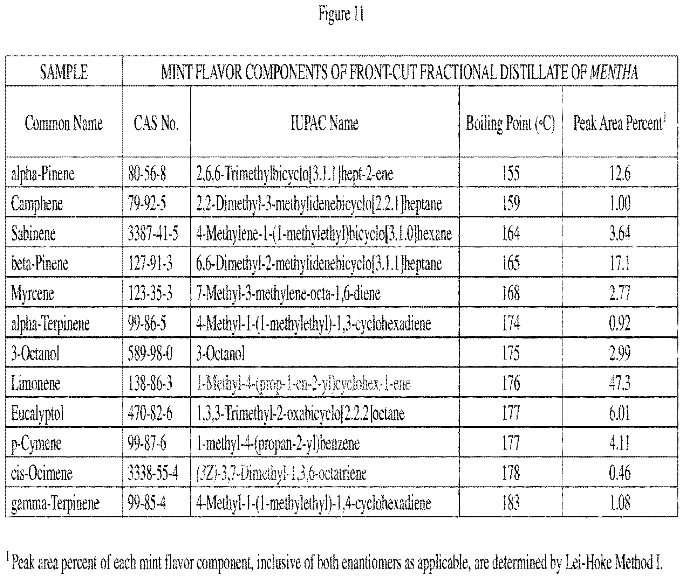

As an example of sample preparation with LHM I is a flavor oil containing a mint flavor composition. Preparation of the flavor oil sample for analysis by LHM I is achieved by pipetting 75-µL of the sample into a 25-mL class A volumetric flask. Dilution to volume with hexane (J.T. Baker, Phillipsburg, NJ, USA) is performed to create a mint flavor composition concentration (from the sample) of 3,000 PPM (v/v). The diluted sample in hexane is then thoroughly mixed by repeated inversion and shaking of the volumetric flask. Inventive examples 1-4 and comparative examples A-O of Figures 1-10 and the "front-cut" fractional distillate example of Figure 11 are prepared for analysis utilizing this procedure.

A second example of sample preparation with LHM I is a dentifrice consumer product containing a mint flavor composition. In this case, a liquid-liquid extraction is utilized, whereby the dentifrice sample is homogenized and dispersed in an aqueous or aqueous salt solution. The resulting aqueous product dispersion is then liquid-liquid extracted with a non-polar solvent, such as hexane. The volume ratio of organic solvent to aqueous product dispersion is optimized so that: the liquid layers are easily separable either with or without centrifugation; the extraction of all flavor components is greater than 90 wt% (relative to the original sample); the concentration of the mint flavor composition (from the sample) is approximately 3,000 PPM (v/v) in the extraction solvent; the largest peak in the GC-MSD total ion chromatogram (TIC) is not saturating the detector; and peaks down to ~0.01 peak area percent relative to the entire mint flavor composition (i.e., including and up to the 37 mint flavor components defined) can be integrated using the total ion chromatogram display. The ratios among dentifrice sample, aqueous dispersing solution, and non-polar extraction solvent are optimized to meet these parameters and to assure a quality sample preparation, consistent with those skilled in the analytical arts, that lead to accurate peak area percent results.

Whether sample preparation is achieved via direct analysis, dilution, or grinding and/or dispersion and/or extraction, in preparation for GC-MSD analysis, the mint flavor composition (from samples) should be contained within a liquid solvent at a concentration of about 3,000 PPM (v/v), when considering the sum of all mint flavor components (i.e., including and up to the 37 mint flavor components defined). After mixing, a ~1.8-mL aliquot is placed into a 2 mL ROBO autosampler vial (VWR International, LLC, Radnor, PA, USA), which is then capped and crimped.

Lei-Hoke Method I: Gas Chromatographic Conditions. The GC injector is configured with a Merlin Microseal (Restek, Bellefonte, PA, USA, part number 22810) with a glass injector liner of dimensions 4 x 6.3 x 78.5mm and containing glass wool (Restek, Bellefonte, PA, USA; part number 20782-213.5). Conditions for the achiral determination of peak area percent for each of the 37 mint flavor components of the mint flavor composition containing samples are: GC inlet temperature is held at 280 °C; the GC is equipped with an Agilent J&W HP FFAP column with dimensions of 30m x 0.25mm ID x 0.25 µm film thickness (Agilent HP FFAP column; part number 19091F-433); the split ratio is 6:1; the carrier gas is helium; the column pressure is -15.7 psi (108.25 kPa); the column flow rate is ~1.15-mL helium/minute; and the GC is run in constant-flow mode throughout the analytical portion of the analysis. For analysis of a given mint flavor composition containing sample, a volume of 1-µL is injected with a 10-µL syringe using a model GC Sampler 80 autosampler (Agilent Technologies, Santa Clara, CA, USA) into the split / splitless GC injector port of an Agilent 7890 gas chromatograph (GC) connected to an Agilent 5975C mass spectrometer detector (MSD).

The GC oven temperature program is held at 40 °C for 1.0 minute, then ramped at 10 °C/minute to 240 °C and held at 240 °C for 5.0 minutes. The GC run time is 26 minutes. The oven temperature is then cooled to 40 °C to prepare for the subsequent injection. Prior to mint flavor composition containing sample analysis, columns are conditioned per manufacturer recommendations and 1-µL organic solvent injections are run, as appropriate, to assure no carry over from previous injections.

The GC analysis conditions for samples prepared by Lei-Hoke Method I are generally applicable to mint flavor composition containing samples with few exceptions. For example, it may be necessary to optimize the GC split ratio to meet the MSD sensitivity requirements or it may be that slowing the temperature ramp to improve chromatographic resolution is needed.

Lei-Hoke Method I: Mass Spectrometer Detector Calibration. Prior to mint flavor composition containing sample analysis, the mass spectrometer is calibrated with FC-43 (Perfluorotributylamine, Agilent; part number GCS-200) in 70 eV electron impact (EI) ionization mode, using the autotune procedure found in Agilent MSD ChemStation (version E.02.02.1431, or equivalent, please see Agilent 5975 Series MSD Operation Manual). Upon completion of the autotune, percent relative abundance (%RA) of key FC-43 ions across the mass calibration range should meet these criteria: m/z 50 (5-25 %RA); m/z 69 (80-100 %RA); m/z 100 (5-25 %RA); m/z 119 (5-20 %RA); m/z 131 (40-60 %RA); m/z 219 (40-100 %RA); m/z 264 (5-30 %RA); m/z 414 (1-15 %RA); and m/z 502 (1-15 %RA). All peaks should be observed at roughly unit mass resolution with peaks full width at half maximum (FWHM) of 0.7 Daltons (Da). All ¹³C isotope peaks should be baseline or nearly baseline resolved from their respective ¹²C isotope peaks. If any of these criteria are not met, the instrument should undergo the appropriate repair, maintenance, troubleshooting and/or recalibration prior to analysis of mint flavor composition containing samples.

Lei-Hoke Method I: Mass Spectrometer Data Acquisition. Effluent from the GC column is directly introduced into the ion source of the 5975C mass spectrometer detector with the following conditions: solvent delay of 4.20 minutes at which time the source filament turns on to begin acquiring mass spectral data; the mass spectrometer transfer line temperature is held at 250 °C; mass spectrometer source temperature is held at 230 °C; and the quadrupole mass analyzer temperature is held at 150 °C. The acquisition range is set to scan from mass to charge ratio (m/z) 33 to 350 at 2 scans per second. The lowest m/z to be scanned must be set above the most abundant air peaks at m/z 28 and m/z 32.

Prior to analysis of mint flavor composition containing samples, some discretion is provided for optimizing mass spectrometer sensitivity. This may be performed via optimizing the GC split ratio and/or sample preparation conditions so that the largest peak representing a mint component found in the mint composition containing sample to be analyzed, usually menthol, should be near linear maximum of the detector response. The largest peak should neither begin to saturate the detector, nor provide a flat-topped peak, such that the MSD response would not correctly measure the peak area percent for the mint flavor component. With these settings, peaks in the total ion chromatogram should be detectable above baseline down to a peak area of ~0.01 percent. If this is not achievable, instrument or method conditions must be optimized as noted above and/or the appropriate repair, cleaning, maintenance, or troubleshooting must be completed to allow the GC-MSD system to meet these criteria prior to obtaining peak area percent data on mint flavor components in mint flavor composition containing samples.

Lei-Hoke Method I: Mass Spectrometer Data Processing. Each mint flavor component of the mint flavor composition containing sample is identified from its retention time and mass spectral fragmentation pattern. As needed, mint flavor component identifications are confirmed via use of reference standard compounds analyzed under the same Lei-Hoke Method I conditions defined above and utilized to analyze the samples. This procedure will confirm the retention time and mass spectra match to a standard and correctly identify a given mint flavor component.

Peaks in the GC-MSD TIC should be evaluated as to whether they are related to a component of the mint flavor composition or not (i.e., does the subject peak belong to one of the 37 mint flavor components). Peaks determined to represent non-mint flavor components are excluded from peak area percent calculations. Examples of peaks that could potentially be observed that should be not included in mint flavor component peak area percent calculations include: (a) flavor components that are not mint flavor components such as methyl salicylate, cinnamaldehyde, vanillin, ethyl vanillin, iso-amyl acetate, benzaldehyde, anethole, etc.; (b) consumer product components and carriers such as humectants like glycerin or propylene glycol; (c) impurities from consumer products such as long chain fatty alcohols or esters that are introduced as impurities from surfactants; (d) impurities from an organic extraction or dilution solvent, such as alkanes that would be observed during analysis of blank injections; and (e) GC-MSD system or background peaks that would also be observed during blank injections. Peak purity should be checked via mass spectral integrity across the peak to assure that there are no co-eluting components (including other mint flavor components). If the peaks are not pure, the situation must be corrected, ideally by optimizing the GC conditions to fully resolve the co-eluting or partially co-eluting components. Peak areas of mint flavor components should then be obtained from the total ion chromatogram for calculation of peak area percents with the following peak area integration parameters: initial threshold 14.5; initial peak width 0.034; shoulder detection OFF; initial area reject 0. When needed, optional manual integration can be utilized, although its use should be minimized, and when used, manual integration must be consistently applied. As above, background, solvent, or other non-mint flavor component peaks should be excluded from the calculation of the area percent of mint flavor composition.

Mint flavor components that should be included in peak area percent determinations are specifically, and up to, the 37 mint flavor components defined. In other words, the collective peak area of these, and up to, 37 mint flavor components, and no other components, is considered 100% peak area percent. It is appreciated that some samples assessed may not have all 37 mint flavor components. In such an event, it is those mint flavor components that are determined to be present in the sample that are collectively considered 100% peak area percent.

Peak area percents for mint flavor components are calculated by summing the total area of, and up to, the 37 mint flavor components identified. The peak area of any one of the 37 mint flavor components assessed is then divided by the total peak area and multiplied by 100 to obtain its achiral peak area percent. Any specific mint flavor component identified (from the 37) is relative to this 100% peak area percent.

Triplicate GC-MSD injections are performed for each mint flavor composition containing sample and reported peak area percents are the average of the results from three separate injections. Those mint flavor components with peak area percents having at least 0.01% are included in the mint flavor composition and calculations of peak area percent. Otherwise, these mint flavor components are excluded because of the threshold of detection limits and minimal impact to the overall determination of 100% peak area percent. Relative standard deviations of peak area percents for each mint flavor component should generally be less than five percent.

### LHM II

Lei-Hoke Method II for Chiral Determination of Peak Area Ratios of Menthol, Menthyl Acetate, Neomenthol, and Isomenthol/Neoisomenthol Enantiomer Pairs in Samples is described. Lei-Hoke Method II is utilized for determination of the relative peak area percent of each enantiomer in each enantiomer pair and the peak area ratio for each enantiomer pair for the following enantiomer pairs in mint flavor compositions (contained within flavors and consumer products): (+)- and (-)-menthol; (+)- and (-)-neomenthol; and (+)- and (-)-menthyl acetate. With this separation, enantiomers of isomenthol and neoisomenthol co-elute and are reported together, i.e. (+)-isomenthol and (+)-neoisomenthol data are combined as well as (-)-isomenthol and (-)-neoisomenthol data are combined. Isomenthol and neoisomenthol enantiomers are well separated from other components including other mint flavor components. Sample preparation conditions for LHM II are the same as specified above for Lei-Hoke Method I: Sample Preparation.

Lei-Hoke Method II: Gas Chromatographic Conditions. The GC conditions for Lei-Hoke Method II differ in critical aspects from Lei-Hoke Methods I, III and IV to allow for GC separation of the specific enantiomer pairs described. The GC injector is configured with a Merlin Microseal (Restek, Bellefonte, PA, USA; part number 22810) with a glass injector liner of dimensions 4 x 6.3 x 78.5mm and containing glass wool (Restek, Bellefonte, PA, USA; part number 20782-213.5). The GC inlet temperature is held at 280 °C and the GC is equipped with a Supelco beta DEX 110 column with dimensions 60m x 0.250mm x 0.25µm film thickness for Lei-Hoke Method II, (Supelco, Bellefonte, PA, USA; part number SU24302). The initial oven temperature is set at 105 °C with a pressure of 35 psi (242.32 kPa), a split ratio of 50:1 and a helium flow rate of 1.6 mL/min. The method is run in constant flow rate mode. Upon injection of a 1-µL sample of mint flavor composition containing sample in organic solvent following preparation as previously described, the GC oven temperature program is held at 105 °C for 80.0 minutes, then ramped at 20 °C /minute to 200 °C and held at 200 °C for 3.25 minutes. The GC run time is 88 minutes. The oven temperature is then cooled to 105 °C to prepare for the subsequent injection. Prior to sample analysis, columns are conditioned per manufacturer recommendations and 1-µL organic solvent blank injections are run, as appropriate, to assure no carry over from previous injections. Reference standard compounds are utilized to confirm the retention time of each enantiomer and baseline, or near baseline separation, of all enantiomer pairs.

Lei-Hoke Method II: Mass Spectrometer Detector Calibration. The MSD calibration for Lei-Hoke Method II is the same as described in detail for Lei-Hoke Method I: Mass Spectrometer Detector Calibration.

Lei-Hoke Method II: Mass Spectrometer Data Acquisition. The MSD data acquisition for Lei-Hoke Method II is the same as described in detail for Lei-Hoke Method I: Mass Spectrometer Data Acquisition with the following exceptions: due to the utilization of a 60-meter column, the solvent delay is set to 8.0 minutes; also, the MSD scan range is modified to m/z 33 - 250.

Lei-Hoke Method II: Mass Spectrometer Data Processing. Each mint flavor component is identified from its retention time and mass spectral fragmentation pattern. As needed, mint flavor component identifications are confirmed via use of reference standard compounds analyzed under the same Lei-Hoke Method II conditions defined above and utilized to analyze mint flavor composition containing samples. This procedure will confirm the retention time and mass spectra match to correctly identify a given compound. Use of reference standard compounds is especially important for the enantiomeric pairs. In cases where pure reference compounds are not readily available, such as (-)-neoisomenthol, (+/-)-neoisomenthol is analyzed as well as (+)-neoisomenthol. The retention time of the (-)-neoisomenthol is determined from the unique peak when comparing these chromatograms and confirmed via EI mass spectrum of neoisomenthol. The sources for the reference standard compounds utilized with this method are: (+)-menthol (TCI (Tokyo Chemical Industry Co., LTD) America, Portland, OR, USA); (-)-menthol (TCI America); (+)-neomenthol (TCI America); (-)-neomenthol (ChemCruz, Santa Cruz, CA, USA); (+)-isomenthol (Sigma-Aldrich, St. Louis, MO, USA); (-)-isomenthol (Sigma-Aldrich); (+)-neoisomenthol (AA Blocks, San Diego, CA, USA); (+/-)-neoisomenthol (ALFA Chemistry, New York, USA); (+)-menthyl acetate (Sigma-Aldrich); and (-)-menthyl acetate (Sigma-Aldrich).

Peak purity is checked via mass spectral integrity across the peaks of interest in LMH II to assure that there are no co-eluting components (including other mint flavor components). If the peaks are not pure, the situation must be corrected, ideally by optimizing the GC conditions to fully resolve the co-eluting components. Peak areas (PA) should then be obtained from manual integration of the total ion chromatogram for each of the peaks in each enantiomer pair specified by LHM II. Manual integration should be consistently applied across peaks. From these data, the peak area percent of each enantiomer in each pair is calculated, for example: % (+)-menthol = PA (+)-menthol / (PA (+)-menthol + PA (-)-menthol) * 100. Additionally, the enantiomer peak area ratio is calculated as, for example: ratio of (+)/(-)-menthol = PA (+)-menthol / PA (-)-menthol. Duplicate GC-MSD injections of mint flavor composition containing samples are performed for each sample and reported peak areas, peak area ratios of enantiomer pairs, and peak area percent of each enantiomer in each enantiomer pair are the average of the results from two separate injections.

### LHM III

Lei-Hoke Method III for Chiral Determination of Peak Area Ratios of Menthone and Isomenthone Enantiomer Pairs in Samples is described. Lei-Hoke Method III is utilized for determination of relative peak area percent of each enantiomer in each enantiomer pair and the peak area ratio for each enantiomer pair for the following enantiomer pairs in mint flavor compositions (contained within flavors and consumer products): (+)- and (-)-menthone; and (+)- and (-)-isomenthone. Sample preparation conditions for this method are the same as specified above for Lei-Hoke Method I: Sample Preparation.

Lei-Hoke Method III: Gas Chromatographic Conditions. The GC conditions for Lei-Hoke Method III differ in critical aspects from Lei-Hoke Methods I, II and IV to allow for GC separation of the specific enantiomer pairs described. The GC injector is configured with a Merlin Microseal (Restek, Bellefonte, PA, USA; part number 22810) with a glass injector liner of dimensions 4 x 6.3 x 78.5mm and containing glass wool (Restek, Bellefonte, PA, USA; part number 20782-213.5). The GC inlet temperature is held at 280 °C; the GC is equipped with a Macherey-Nagel Lipodex E column with dimensions 25m x 0.250mm (film thickness is not available from the column manufacture, Macherey-Nagel GmbH & Co., Duren, Germany; part number 723368.25). The initial oven temperature is set at 100 °C with a pressure of 16.5 psi (113.76 kPa), a split ratio of 50:1 and a helium flow rate of 1.1 mL/min. The method is run in constant flow rate mode. Upon injection of a 1-µL of mint flavor composition containing sample in organic solvent following preparation as described, the GC oven temperature program is held at 100 °C for 12.0 minutes, then ramped at 20 °C / minute to 200 °C and held at 200 °C for 3.0 minutes. The GC run time is 20 minutes. The oven temperature is then cooled to 100 °C to prepare for the subsequent injection. Prior to sample analysis, columns are conditioned per manufacturer recommendations and 1-µL organic solvent blank injections are run, as appropriate, to assure no carry over from previous injections. Reference standard compounds are utilized to confirm the retention time of each enantiomer and baseline or near baseline separation of all enantiomer pairs.

Lei-Hoke Method III: Mass Spectrometer Detector Calibration. The MSD calibration for Lei-Hoke Method III is the same as described in detail for Lei-Hoke Method I: Mass Spectrometer Detector Calibration.

Lei-Hoke Method III: Mass Spectrometer Data Acquisition. The MSD data acquisition for Lei-Hoke Method III is the same as described in detail for Lei-Hoke Method I: Mass Spectrometer Data Acquisition except for the use of a solvent delay time of 5.0 minutes.

Lei-Hoke Method III: Mass Spectrometer Data Processing. Each mint flavor component is identified from its retention time and mass spectral fragmentation pattern. As needed, mint flavor component identifications are confirmed via use of reference standard compounds analyzed under the same Lei-Hoke Method III conditions defined and utilized to analyze mint flavor composition containing samples. This procedure will confirm the retention time and mass spectra match to correctly identify a given compound. Use of reference standard compounds is especially important for the enantiomeric pairs. The sources for the reference standard compounds utilized with this method are: (+)-menthone (Sigma-Aldrich, St. Louis, MO, USA); (-)-menthone (Sigma-Aldrich); (+)-isomenthone (AA Blocks, San Diego, CA, USA); and (-)-isomenthone (AA Blocks).

Peak purity is checked via mass spectral integrity across the peaks of interest in LMH III to assure that there are no co-eluting components (including other mint flavor components). If the peaks are not pure, the situation must be corrected, ideally by optimizing the GC conditions to fully resolve the co-eluting components. Peak areas (PA) should then be obtained from manual integration of the total ion chromatogram for each of the peaks in each enantiomer pair specified by LHM III. Manual integration should be consistently applied across peaks. From these data, the peak area percent of each enantiomer in each enantiomer pair can be calculated, for example: % (+)-menthone = PA (+)-menthone / (PA (+)-menthone + PA (-)-menthone) * 100. Additionally, the enantiomer peak area ratio can be calculated, for example: ratio of (+)/(-)-menthone = PA (+)-menthone / PA (-)-menthone. Duplicate GC-MSD injections of mint flavor composition containing samples are performed for each sample and reported peak areas, peak area ratios of enantiomer pairs, and peak area percent of each enantiomer in each enantiomer pair are the average of the results from two separate injections.

### LHM IV

Lei-Hoke Method IV for Chiral Determination of Peak Area Ratios of Seven Enantiomer Pairs of Mint Flavor Components in Samples is described. Lei-Hoke Method IV is utilized for determination of relative peak area percent of each enantiomer in each enantiomer pair and the peak area ratio for each enantiomer pair for the following enantiomer pairs in mint flavor compositions (contained within flavors and consumer products): (+)- and (-)-alpha-pinene; (+)- and (-)-beta-pinene; (+)- and (-)-limonene; (+)- and (-)-linalool; (+)- and (-)-isopulegol; (+)- and (-)-terpinen-4-ol; and (+)- and (-)-piperitone. Sample preparation conditions for this method are the same as specified above for Lei-Hoke Method I: Sample Preparation.

Lei-Hoke Method IV: Gas Chromatographic Conditions. The GC conditions for Lei-Hoke Method IV differ in critical aspects from Lei-Hoke Methods I, II and III to allow for GC separation of the specific enantiomer pairs described above. The GC injector is configured with a Merlin Microseal (Restek, Bellefonte, PA, USA; part number 22810) with a glass injector liner of dimensions 4 x 6.3 x 78.5mm and containing glass wool (Restek, Bellefonte, PA, USA; part number 20782-213.5). The GC inlet temperature is held at 280 °C; the GC is equipped with an Agilent HP 20B Chiral column with dimensions 30m x 0.25mm x 0.25µm film thickness (Agilent part number 19091G-B233). The initial oven temperature is set at 40 °C with a pressure of 15.7 psi (108.25 kPa), a split ratio of 10:1 and a helium flow rate of 1.14 mL/min. The method is run in constant flow rate mode. Upon injection of a 1-µL sample of mint flavor composition containing sample in organic solvent following preparation as described, the GC oven temperature program is held at 40 °C for 2.0 minutes, then ramped at 4 °C /min to 220 °C and held at 220 °C for 1.0 minute. The GC run time is 48 minutes. The oven temperature is then cooled to 40 °C to prepare for the subsequent injection. Prior to sample analysis, columns are conditioned per manufacturer recommendations and 1-µL organic solvent blank injections are run, as appropriate, to assure no carry over from previous injections. Standard compounds are utilized to confirm the retention time of each enantiomer and baseline or near baseline separation of all enantiomer pairs.

Lei-Hoke Method IV: Mass Spectrometer Detector Calibration. The MSD calibration for Lei-Hoke Method IV is the same as described in detail for Lei-Hoke Method I: Mass Spectrometer Detector Calibration.

Lei-Hoke Method IV: Mass Spectrometer Data Acquisition. The MSD data acquisition for Lei-Hoke Method IV is the same as described in detail for Lei-Hoke Method I: Mass Spectrometer Data Acquisition.

Lei-Hoke Method IV: Mass Spectrometer Data Processing. Each mint flavor component is identified from its retention time and mass spectral fragmentation pattern. As needed, mint flavor component identifications are confirmed via use of reference standard compounds analyzed under the same Lei-Hoke Method IV conditions defined and utilized to analyze mint flavor composition containing samples. This procedure will confirm the retention time and mass spectra match to correctly identify a given compound. Use of reference standard compounds is especially important for the identification of enantiomeric pairs because of their very close retention time and similar mass spectra. In cases where pure reference standard compounds are not readily available, such as (+)-linalool, (-) linalool is analyzed as well as (+/-) linalool. The retention time of the (+)-linalool is determined from the unique peak when comparing these chromatograms and confirmed via the EI mass spectrum of linalool. The sources for the reference standard compounds utilized with this method are: (+)-alpha-pinene (TCI (Tokyo Chemical Industry Co., LTD) America, Portland, OR, USA); (-)-alpha-pinene (TCI America); (+)-beta-pinene (AA Blocks, San Diego, CA, USA); (-)-beta-pinene (Sigma-Aldrich, St. Louis, MO, USA); (+)-limonene (TCI America); (-)-limonene (TCI America); (-)-linalool (Sigma-Aldrich); (+/-)-linalool (AA Blocks); (-)-terpinen-4-ol (Sigma-Aldrich); (+/-)- terpinen-4-ol (AA Blocks); (-)-piperitone (Atlantic Research Chemicals Ltd, Cornwall, United Kingdom); racemic piperitone (mixtures of enantiomers, predominantly (R)-(-)-form, TCI America); (+)-isopulegol (Sigma-Aldrich); and (-)-isopulegol (Sigma-Aldrich).

Peak purity is checked via mass spectral integrity across the peaks of interest in LMH IV to assure that there are no co-eluting components (including other mint flavor components). If the peaks are not pure, the situation must be corrected, ideally by optimizing the GC conditions to fully resolve the co-eluting components. Peak areas (PA) should then be obtained from manual integration of the total ion chromatogram for each of the peaks in each enantiomer pair specified by LHM IV. Manual integration should be consistently applied across peaks. From these data, the peak area percent of each enantiomer in the pair can be calculated, for example: % (+)-linalool = PA (+)-linalool / (PA (+)-linalool + PA (-)-linalool) * 100. Additionally, the enantiomer peak area ratio can be calculated, for example: ratio of (-)/(+)-linalool = PA (-)-linalool / PA (+)-linalool. Duplicate GC-MSD injections of mint flavor composition containing samples are performed for each sample and reported peak areas, ratios of enantiomer pairs, and percent of each enantiomer in each enantiomer pair are the average of the results from two separate injections.

### LHM V

Lei-Hoke Method V for Calculation of Peak Area Percent of Individual Mint Component Enantiomers in Samples is described. With Lei-Hoke method I, the peak area percent of each mint flavor component in a mint flavor composition containing sample is determined, with respective enantiomers measured together. Using menthol as an example, achiral Lei-Hoke method I measures the combined response and peak area for (+)-menthol and (-)-menthol in a mint flavor composition containing sample, then calculates and reports the peak area percent as menthol. With Lei-Hoke methods II-IV, peak area percents of each enantiomer within each enantiomer pair, and/or enantiomeric peak area ratios, within key mint flavor component enantiomer pairs are determined. From the data obtained in LHM I and the appropriate data for a given enantiomer pair obtained from LHM II-IV, Lei-Hoke method V details the procedures to calculate the peak area percent composition of each enantiomer in a mint flavor composition containing sample using the following formulas: % of the (-)-enantiomer in a mint flavor composition = (achiral % in mint flavor composition from LHM I) * (% (-)-enantiomer in the enantiomer pair from LHM II, III or IV/100); likewise, % of the (+)-enantiomer in a mint flavor composition = (achiral % in mint flavor composition from LHM I) * (% (+)-enantiomer in the enantiomer pair from LHM II, III or IV/100).

As a hypothetical example for illustrative purposes, upon analysis of a mint flavor composition containing sample via Lei-Hoke Method I, menthol is determined to be 50 peak area percent of the overall mint flavor composition. The sample is also analyzed by Lei-Hoke method II, from which the peak area of (+)-menthol is determined to be 1,000 area units and the peak area of (-)-menthol is determined to be 10,000 area units. From LHM II, the corresponding peak area percent of (+)-menthol in the menthol enantiomer pair is ((1,000)/(1,000 +10,000))*100 = 9.09%. Likewise, from LHM II, the corresponding peak area percent of (-)-menthol in the menthol enantiomer pair is ((10,000)/(1,000+10,000))*100 = 90.9%. From Lei-Hoke Method V, it is further calculated that the peak area percent of (+)-menthol in the mint flavor composition = 50% * (9.09%/100) = 4.55% and the % of (-)-menthol in the mint flavor composition = 50% *(90.9%/100) = 45.5%.

### Mint Flavor Compositions

The mint flavor compositions of the present invention comprise one or more of the following mint flavor components (and one or more additional mint flavor components):

### Menthol

An aspect of the invention provides for a mint flavor composition comprising: a mint flavor component that is racemic menthol and an additional mint flavor component, preferably a combination of racemic menthol and (-)-menthol, more preferably a combination of racemic menthol and (-)-menthol while minimizing the amount of neomenthol, isomenthol, and neoisomenthol. The combination provides the benefits of a cooling sensation, minimizing negatives from less desirable stereoisomers, while being cost effective. Menthol has three chiral centers, and thus has eight stereoisomers, specifically (+)-menthol, (+)-isomenthol, (+)-neomenthol, (+)-neosiomenthol, (-)-menthol, (-)-isomenthol, (-)-neomenthol, and (-)-neoisomenthol. Natural menthol primarily exists as the (1*R,* 2*S*, 5*R*)-stereoisomer form, also known as (-)-menthol, accounting for perhaps 35 - 50% of the aroma chemicals present in natural peppermint oil. Other isomers of menthol (i.e., neomenthol, isomenthol and neoisomenthol) have somewhat similar, but not identical odor and taste, i.e., some having disagreeable notes described, from internal unpublished research, as earthy, camphor, musty, motor oil, shoe leather, and burnt rubber. The principal difference among the isomers is in their cooling potency. (-)-Menthol provides the most potent cooling. However, synthetic (-)-menthol is more expensive than racemic menthol.

A more cost-effective approach is the use of racemic menthol, or preferably substituting a portion of (-)-menthol in the mint flavor composition with racemic menthol. A partial replacement helps provide the desirable cooling sensation that many consumers come to expect in a high-quality mint profile, but notably saves costs. Racemic menthol is also known as a 50:50 (-)-menthol: (+)-menthol mixture or DL-menthol. Even more preferably, is minimizing the amount of total neomenthol/isomenthol/neoisomenthol given some of the negative sensory/taste characteristics that accompany these stereoisomers. Generally, the mint flavor compositions herein employ a higher level of non-natural enantiomers and/or ratios to minimize cost and optimize the flavor profile by carefully balancing these levels and/or ratios.

An aspect of the invention provides for a mint flavor composition comprising: a mint flavor component that is menthol and an additional mint flavor component. Preferably (+)- and (-)-menthol has a peak area percent of 40.0 - 45.0, preferably 41.5 - 45.0, alternatively 42.0 - 43.5, as determined by Lei-Hoke Method I. Preferably a peak area ratio of (+)-menthol : (-)-menthol is from 0.2 - 0.4, preferably 0.21 - 0.35, alternatively 0.30 - 0.34, or 0.220 - 0.319, or 0.3 - 0.4, as determined by Lei-Hoke Method II. The (+)-menthol may have a peak area percent from 6 - 12, preferably 7.0 - 11.0, alternatively from 9.5 - 10.5, as determined by Lei-Hoke Method V. The (-)-menthol may have a peak area percent from 30 - 37, preferably 31.0 - 36.0, alternatively from 31.5 - 32.5, as determined by Lei-Hoke Method V. Racemic menthol may have a peak area percent from 14 - 22, preferably 15.0 - 21.0, alternatively from 19.5 - 20.5, as determined by Lei-Hoke Method V. Non-racemic (-)-menthol may have a peak area percent from 5 - 29.0, preferably 15 - 28.5, more preferably 19 - 28.0, alternatively 20 - 28.0 or 19.0 - 23.0, as determined by Lei-Hoke Method V. Preferably a peak area ratio of racemic menthol : non-racemic (-)-menthol is from 0.5 - 1, preferably 0.7 - 1, alternatively from 0.8 - 1 or 0.900 - 0.950, as determined by Lei-Hoke Method V. Suitable mint flavor compositions, as described above in reference to peak area percent, can be additionally represented in wt% of the mint flavor composition. Thus, suitable mint flavor compositions can comprise from about 35% to about 45%, from about 30% to about 50%, or from about 35% to about 50%, by weight of the mint flavor composition of racemic menthol, (-)-menthol, (+)-menthol, and/or combinations thereof.

An aspect of the invention provides for a mint flavor composition comprising: mint flavor components that are neomenthol, isomenthol, and/or neoisomenthol; and an additional mint flavor component. Generally, the mint flavor compositions have less neomenthol, isomenthol, and/or neoisomenthol than the comparative examples assessed, which is indicative of the synthetic nature of the composition and deemphasizing less preferred flavor notes. Preferably the mint flavor composition comprises (+)-neomenthol, wherein the (+)-neomenthol has a peak area percent from 0.2 - 1.5, preferably 0.4 - 1, alternatively from 0.5 - 0.7, as determined by Lei-Hoke Method V. Preferably the mint flavor composition comprises (+)- and (-)-isomenthol, wherein the (+)- and (-)-isomenthol has a peak area percent from 0.1 - 0.3, preferably 0.11 - 0.25, alternatively 0.14 - 0.23, as determined by Lei-Hoke Method I. The composition may comprise neoisomenthol, wherein the neoisomenthol has a peak area percent from 0.01 - 0.2, preferably 0.02 - 0.18, alternatively 0.02 - 0.05, as determined by Lei-Hoke Method I. Preferably the mint flavor compositions minimize the total content of neomenthol, isomenthol, and/or neoisomenthol. To this end, the mint flavor composition may comprise less than 3.5, preferably 0.01 - 2.2, more preferably 0.1 - 2, even more preferably 0.2 - 1.8, alternatively 0.1 - 3.5 or 0.5 - 3.0 or 1 - 2, peak area percent in total content of neomenthol, isomenthol, and/or neoisomenthol, as determined by Lei-Hoke Method I. The mint flavor compositions may comprise menthol and neomenthol, wherein a peak area ratio of menthol : neomenthol is from 19 - 80, preferably 25 - 60, more preferably 30 - 55, as determined by Lei-Hoke Method I.

The following data helps support the use of racemic menthol to reduce costs of mint flavor compositions herein described. From internal unpublished research, Table B compares replacing (-)-menthol with a racemic menthol in a 1:1 in a toothpaste formulation. Such a direct replacement is not preferred given the reduced cooling profile and olfactory differences; however, a portion of L-menthol is preferably replaced by racemic-menthol to gain cost efficiency while minimizing impact to the overall mint flavor profiles.

**Table B is a comparison of attributes between (-)-menthol and racemic menthol in a toothpaste context (using CREST Cavity formulation).**

| Attribute | (-)-Menthol | Racemic Menthol |
|---|---|---|
| Maximum Cooling (0-60 scale¹) | 40 | 35 |
| Time Point at Maximum Cooling (minutes) | ~5 | ~1 |
| Maximum Longevity (minutes) | Up to 25 | Up to 20 |
| EC 50² (parts per million) | 1,750 to 2,250 | 1,250 to 1,500 |
| Potency compared to L-Menthol | 1x | ~0.65-0.7x |
| Cost compared to L-Menthol | 1x | ~0.5x |
| Flavor Profile | Clean, minty, sweet | Distracting notes at high concentrations |

| | | |
|---|---|---|
| ¹ 60 is defined as the greatest amount of cooling whereas 0 is the least amount of cooling. ²EC 50 is the half maximal Effective Concentration referring to the concentration of the coolant material which induces a response halfway between the baseline and maximum cooling. This value represents the concentration of a coolant where 50% of its maximal cooling is observed. | | |

In separate, unpublished internal experiments, perceptual experiences of racemic menthol are quantified via expert sensory testing. The use of a standard spearmint flavor (minimizing extra menthol contribution) compared racemic menthol to (-)-menthol in a toothpaste formulation (CREST Cavity). There are several sensory observations taken from these experiments. Firstly, racemic menthol is about 25-30% less potent than (-)-menthol for cooling. At equivalent concentration, (-)-menthol is perceived as colder, more minty, less bitter, less drying and delivers a high overall clean mouth sensation. Secondly, at higher concentration (i.e., greater than 5,000 part per million), racemic menthol is characterized as pencil lead, burnt rubber, shoe rubber/leather and motor oil. These aromatic notes appear during brushing and disappear after 5-10 minutes post expectoration and are more pronounced as concentrations increase. Thirdly, there are no meaningful differences in multiple attributes (peppery burn, cooling vapors, thermal diffusion and cold). Lastly, there are no meaningful differences detected in the oral cavity for all sensory attributes measured.

### Menthone

An aspect of the invention provides for a mint flavor composition comprising: a mint flavor component that is menthone, and an additional mint flavor component. Generally, the mint flavor compositions herein employ a higher level of non-natural enantiomers and/or ratios to minimize cost and optimize the flavor profile by carefully balancing these levels and/or ratios. Preferably the menthone has a peak area percent of 21 - 26, preferably 22.0 - 26.0, alternatively 21.5 - 23.5 or 22.0 - 23.0, as determined by Lei-Hoke Method I. Preferably a peak area ratio of the (+)-menthone: (-)-menthone is from 0.9 - 1, preferably 0.91 - 0.99, as determined by Lei-Hoke Method III. Without wishing to be bound by theory, the aroma profiles of (-)-, (+)- and racemic menthone are similar, though (+)- and racemic menthone have slightly more earthy/musty notes than the (-)-form. The mint flavor composition may comprise menthol and menthone, wherein a peak area ratio of menthol : menthone is from 1.6 - 2, preferably 1.7 - 1.9, as determined by Lei-Hoke Method I. In combination with the addition of racemic menthol (described above), a significant percentage of the overall flavor mint composition is represented by the inclusion of these menthol and menthone components, and accordingly there is a significant impact to the overall flavor profile and thus cost savings achieved from the balancing of less expensive racemics while accounting for the level of contribution of negative aromatics.

The mint flavor composition may also comprise isomenthone. The isomenthone may have a peak area percent from 5 - 10, preferably 5.2 - 9, alternatively 7.5 - 8.5, as determined by Lei-Hoke Method I. Preferably a peak area ratio of (-)-isomenthone : (+)-isomenthone is from 0.850 - 0.999, preferably 0.90 - 0.98, as determined by Lei-Hoke Method III. (-)-Isomenthone exhibits a vegetative, beany-like character whereas (+)- isomenthone and racemic bring in a more pungent aroma such as horseradish and vinegar. While (-)-isomenthone is preferred for building substantivity in a mint flavor aroma profile, low levels of (+)-isomenthone and racemic may bring in lift and nasal impact due to the pungent character.

Suitable mint flavor compositions including menthone and/or isomenthone, as described above in reference to peak area percent, can be additionally represented in wt% of the mint flavor composition. Thus, suitable mint flavor compositions can comprise from about 22% to about 26%, from about 20% to about 30%, or from about 20% to about 27%, by weight of the mint flavor composition of racemic menthone, (-)-menthone, (+)-menthone, racemic isomenthone, (-)-isomenthone, (+)-isomenthone, and/or combinations thereof.

### Menthyl Acetate

An aspect of the invention provides for a mint flavor composition comprising: a mint flavor component that is menthyl acetate and an additional mint flavor component. Generally, the mint flavor compositions herein employ a higher level of non-natural enantiomers and/or ratios to minimize cost and optimize the flavor profile by carefully balancing these levels and/or ratios. Preferably the menthyl acetate has a peak area percent from 5.5 - 6.5, preferably 5.8 - 6.5, alternatively 6.0 - 6.3, as determined by Lei-Hoke Method I. Preferably a peak area ratio of (+)-menthyl acetate : (-)-menthyl acetate is from 0.1 - 0.980, preferably 0.7 - 0.980, alternatively 0.900 - 0.980, as determined by Lei-Hoke Method II. Without wishing to be bound by theory, menthyl acetate imparts a characteristic peppermint note coupled with a sweet, ethereal, cedar and woody character. While racemic menthyl acetate is slightly less impactful than (-)-menthyl acetate, their aroma profiles are extremely similar and using a racemic blend within a mint flavor composition reduces cost without bringing in negative attributes.

Suitable mint flavor compositions including menthyl acetate, as described above in reference to peak area percent, can be additionally represented in wt% of the mint flavor composition. Thus, suitable mint flavor compositions can comprise from about 1% to about 12%, from about 0.01% to about 15%, or from about 0.1% to about 12%, by weight of the mint flavor composition of racemic menthyl acetate, (-)-menthyl acetate, (+)-menthyl acetate, and/or combinations thereof.

### Dihydromint Lactone

An aspect of the invention provides for a mint flavor composition comprising: a mint flavor component that is dihydromint lactone and an additional mint flavor component. Preferably the dihydromint lactone is from 0.035 - 0.500, preferably 0.040 - 0.300, more preferably 0.045 - 0.100 of peak area percent, as determined by Lei-Hoke Method I. Without wishing to be bound by theory, the addition of dihydromint lactone is important because it imparts a dairy-like creaminess, enhanced mint body and fullness reminiscent of a natural mint composition. There is not any significant amount of dihydromint lactone in the commercially available mint compositions assessed. That is, inventive examples contained higher levels of dihydromint lactone as compared to the comparative examples. Preferably, dihydromint lactone is sourced from synthetic sources given cost advantages.

Suitable mint flavor compositions including dihydromint lactone, as described above in reference to peak area percent, can be additionally represented in wt% of the mint flavor composition. Thus, suitable mint flavor compositions can comprise from about 0.035% to about 0.500%, from about 0.025% to about 0.750%, or from about 0.1% to about 12%, by weight of the mint flavor composition of racemic dihydromint lactone, (-)-dihydromint lactone, (+)-dihydromint lactone, and/or combinations thereof.

### alpha-Pinene

An aspect of the invention provides for a mint flavor composition comprising: a mint flavor component that is alpha-pinene and an additional mint flavor component. Generally, the mint flavor compositions herein employ a higher level of non-natural enantiomers and/or ratios to minimize cost and optimize the flavor profile by carefully balancing these levels and/or ratios. Preferably the alpha-pinene has a peak area percent from 1.90 - 5, preferably 2.00 - 4, more preferably 2.20 - 3.5, as determined by Lei-Hoke Method I. Preferably a peak area ratio of (-)-alpha-pinene : (+)-alpha-pinene is from 3.0 - 6, preferably 3.1 - 5, more preferably 3.2 - 4.7, alternatively 3.5 - 4.5, as determined by Lei-Hoke Method IV. (-)-alpha-Pinene may have a peak area percent from 1.5 - 2.5, as determined by Lei-Hoke Method V. (+)-alpha-Pinene may have a peak area percent from 0.40 - 0.60, as determined by Lei-Hoke Method V. Without wishing to be bound by theory, (-)- and (+)-alpha-pinene isomers exhibit some of the largest aromatic differences from other components. From an expert flavorist's perspective, the (-)-form is described as animalic and sweaty while the (+)-form is reminiscent of a green apple. In this case, the heavier animalic notes of the (-)-alpha-pinene are preferred for the character profile to enhance the richness and body, whereas the light apple notes of the (+)-alpha-pinene are too ethereal and fleeting.

Suitable mint flavor compositions including alpha-pinene, as described above in reference to peak area percent, can be additionally represented in wt% of the mint flavor composition. Thus, suitable mint flavor compositions can comprise from about 1% to about 5%, from about 0.01% to about 10%, or from about 0.1% to about 5%, by weight of the mint flavor composition of alpha-pinene, racemic alpha-pinene, (-)-alpha-pinene, (+)-alpha-pinene, and/or combinations thereof.

### beta-Pinene

An aspect of the invention provides for a mint flavor composition comprising: a mint flavor component that is beta-pinene, preferably at least (-)-beta-pinene; and an additional mint flavor component. Generally, the mint flavor compositions herein employ a higher level of non-natural enantiomers and/or ratios to minimize cost and optimize the flavor profile by carefully balancing these levels and/or ratios. Preferably a peak area percent of (-)-beta-pinene is from 1.1 - 5, preferably 1.2 - 3, more preferably 1.5 - 2.5, alternatively 2.0 - 2.4, as determined by Lei-Hoke Method V. Preferably the beta-pinene has a peak area percent from 2.2 - 5.0, preferably 2.3 - 4.0, preferably 2.4 - 3.0, as determined by Lei-Hoke Method I. The composition may have a peak area ratio of (-)-beta-pinene : (+)-beta-pinene from 3 - 8, preferably 4 - 7, more preferably 4.7 - 6.0, as determined by Lei-Hoke Method IV. Without wishing to be bound by theory, beta-pinene may impart greater levels of green, pine-like woody notes to the mint flavor compositions herein.

Suitable mint flavor compositions including beta-pinene, as described above in reference to peak area percent, can be additionally represented in wt% of the mint flavor composition. Thus, suitable mint flavor compositions can comprise from about 0.5% to about 3%, from about 0.01% to about 3%, or from about 0.1% to about 5%, by weight of the mint flavor composition of beta-pinene, racemic beta-pinene, (-)-beta-pinene, (+)-beta-pinene, and/or combinations thereof.

### Limonene

An aspect of the invention provides for a mint flavor composition comprising: a mint flavor component that is limonene and an additional mint flavor component. Preferably a peak area percent of limonene is from 3.80 - 8, preferably 4.00 - 7, more preferably 4.30 - 6.50, alternatively 4.0 - 5.5 or 4.50 - 5.50, as determined by Lei-Hoke Method I. Preferably a peak area ratio of (-)-limonene : (+)-limonene, is from 5 to 40, preferably from 11 - 35, as determined by Lei-Hoke Method IV. Preferably a peak area percent of (-)-limonene is from 4.00 - 7, preferably 4.30 - 6 of peak area percent, as determined by Lei-Hoke Method V. (+)-Limonene may have a peak area percent of 0.100 - 0.500, alternatively 0.400 - 0.500, as determined by Lei-Hoke Method V. Without wishing to be bound by theory, (-)-limonene is the configuration most commonly associated with mint due to its terpeney, piney aroma. (+)-Limonene exhibits more floral, citrus notes, and even racemic limonene connotes "peely," citrus character. Therefore, (-)-limonene is the preferred isomer for the mint flavor compositions herein, as citrus notes may skew the aroma flavor profile in a different direction.

Suitable mint flavor compositions including limonene, as described above in reference to peak area percent, can be additionally represented in wt% of the mint flavor composition. Thus, suitable mint flavor compositions can comprise from about 2.40% to about 8.00%, from about 2% to about 10%, or from about 2.50% to about 7.50%, by weight of the mint flavor composition of limonene, racemic limonene, (-)-limonene, (+)-limonene, and/or combinations thereof.

### Linalool

An aspect of the invention provides for a mint flavor composition comprising: a mint flavor component that is linalool, preferably (-)-linalool; and an additional mint flavor component. Generally, the mint flavor compositions herein employ a higher level of non-natural enantiomers and/or ratios to minimize cost and optimize the flavor profile by carefully balancing these levels and/or ratios. Preferably the (-)-linalool has a peak area percent from 0.117 - 0.2, preferably 0.120 - 0.200, more preferably 0.125 - 0.190, alternatively 0.125 - 0.185, as determined by Lei-Hoke Method V. Preferably linalool has a peak area percent from 0.22 - 0.40, preferably 0.22 - 0.35, more preferably 0.25 - 0.28, alternatively 0.260 - 0.270, as determined by Lei-Hoke Method I. Preferably a peak area ratio of (-)-linalool : (+)-linalool is from 0.5 - 2.5, preferably 0.9 - 2.3, as determined by Lei-Hoke Method IV. Without wishing to be bound by theory, linalool brings a green, floral character to the overall mint flavor composition, while using a racemic form of linalool is more cost effective.

Suitable mint flavor compositions including linalool, as described above in reference to peak area percent, can be additionally represented in wt% of the mint flavor composition. Thus, suitable mint flavor compositions can comprise from about 0.12% to about 0.40%, from about 0.10% to about 0.50%, or from about 0.15% to about 0.65%, by weight of the mint flavor composition of linalool, racemic linalool, (-)-linalool, (+)-linalool, and/or combinations thereof.

### Thymol

An aspect of the invention provides for a mint flavor composition comprising: a mint flavor component that is thymol and an additional mint flavor component. Preferably the thymol is 0.03 - 0.15, preferably 0.05 - 0.10, of peak area percent, as determined by Lei-Hoke Method I. Without wishing to be bound by theory, thymol contributes an impactful, camphoraceous character to mint flavor compositions.

Suitable mint flavor compositions including thymol, as described above in reference to peak area percent, can be additionally represented in wt% of the mint flavor composition. Thus, suitable mint flavor compositions can comprise from about 0.03% to about 0.15%, from about 0.01% to about 0.20%, or from about 0.02% to about 0.25%, by weight of the mint flavor composition of thymol.

### Eucalyptol

An aspect of the invention provides for a mint flavor composition comprising a mint flavor component that is eucalyptol and an additional mint flavor component. Preferably the eucalyptol is from 3 - 5.5, preferably 3.5 - 5, alternatively 3.8 - 4.5, of peak area percent, as determined by Lei-Hoke Method I. Without wishing to be bound by theory, eucalyptol is impactful and uplifting to the overall flavor profile. It may also help carry other components, but too much may impart an undesirable medicinal taste to the flavor profile.

Suitable mint flavor compositions including eucalyptol, as described above in reference to peak area percent, can be additionally represented in wt% of the mint flavor composition. Thus, suitable mint flavor compositions can comprise from about 2.3% to about 6.0%, from about 2.0% to about 7.5%, or from about 1% to about 5%, by weight of the mint flavor composition of eucalyptol.

### Menthofuran

An aspect of the invention provides for a mint flavor composition comprising: a mint flavor component that is menthofuran and an additional mint flavor component. Generally, the mint flavor compositions herein have less menthofuran than the comparative examples assessed, which is indicative of the synthetic nature of the composition and deemphasizing less preferred flavor notes. Preferably a peak area percent of menthofuran is from 0.01 - 0.10, alternatively 0.04 - 0.08, as determined by Lei-Hoke Method I. The mint flavor composition may comprise menthyl acetate and menthofuran, wherein a peak area ratio of menthyl acetate : menthofuran is from 60 - 225, preferably 61 - 200, more preferably 62 - 185, alternatively 80 - 130, as determined by Lei-Hoke Method I. The mint flavor composition may also comprise eucalyptol and menthofuran, wherein a peak area ratio of eucalyptol : menthofuran is from 40 - 115, alternatively 50 - 90, as determined by Lei-Hoke Method I. The composition may yet also comprise menthyl acetate, eucalyptol, and menthofuran, wherein a peak area ratio of menthyl acetate : menthofuran is from 60 - 225, preferably 61 - 200, more preferably 62 - 185, as determined by Lei-Hoke Method I, and a peak area ratio of eucalyptol : menthofuran is from 40 - 115, as determined by Lei-Hoke Method I.

### Caryophyllene

An aspect of the invention provides for a mint flavor composition comprising: a mint flavor component and optionally caryophyllene as an additional mint flavor component. Preferably the caryophyllene is from 0 - 0.30, alternatively 0.08 - 0.16, of peak area percent, as determined by Lei-Hoke Method I.

### Carvone

An aspect of the invention provides for a mint flavor composition comprising: a mint flavor component and carvone as an additional mint flavor component. Preferably the carvone is from 0.05 - 0.20, alternatively 0.06 - 0.12, of peak area percentage, as determined by Lei-Hoke Method I.

### Piperitone

An aspect of the invention provides for a mint flavor composition comprising: a mint flavor component that is piperitone and an additional mint flavor component. Preferably the piperitone is 0.1 - 1.0, preferably 0.2 - 0.7, alternatively 0.3 - 0.55 or 0.4 - 0.6, peak area percent, as determined by Lei-Hoke Method I. Preferably the mint flavor composition comprises a peak area ratio of (-)-piperitone : (+)-piperitone from 2 - 18, preferably 5- 15, alternatively from 12 - 16, as determined by Lei-Hoke Method IV.

### Terpinen-4-ol

An aspect of the invention provides for a mint flavor composition comprising: a mint flavor component and optionally terpinen-4-ol as an additional mint flavor component. Preferably the terpinen-4-ol is 0 to 0.5, preferably 0 - 0.3, peak area percent, as determined by Lei-Hoke Method I.

### Isopulegol

An aspect of the invention provides for a mint flavor composition comprising: a mint flavor component and isopulegol as an additional mint flavor component. Preferably the isopulegol is from 0.20 - 0.60, preferably 0.21 - 0.50, peak area percent, as determined by Lei-Hoke Method I.

### Viridiflorol

An aspect of the invention provides for a mint flavor composition comprising: a mint flavor component that is viridiflorol and an additional mint flavor component. Preferable the viridiflorol is from 0.01 - 0.2, preferably 0.02 - 0.08, alternatively 0.03 - 0.06, peak area percent, as determined by Lei-Hoke Method I.

### p-Cymene, Pulegone, alpha-Terpineol, 3-Hexen-1-ol

An aspect of the invention provides for a mint flavor composition comprising: a mint flavor component and an additional mint flavor component selected from p-cymene, pulegone, alpha-terpineol, 3-hexen-1-ol, and combinations thereof. When present, the composition may comprise, for example, from: 0.310 - 0.390 peak area percent of p-cymene; 0.050 - 0.270 peak area percent of pulegone; 0.090 - 0.110 peak area percent of alpha-terpineol; 0.01 - 0.1, preferably 0.01 - 0.05, more preferably 0.01 - 0.03 peak area percent of 3-hexen-1-ol; and combinations thereof, as determined by Lei-Hoke Method I. Without wishing to be bound by theory, 3-hexen-1-ol may be used to impart a fresh green leafy mint note.

### Monoterpenes

An aspect of the invention provides for a mint flavor composition comprising: mint flavor components that are C₁₀H₁₆ monoterpenes and an additional mint flavor component. The C₁₀H₁₆ monoterpenes are selected from the group consisting of sabinene, myrcene, camphene, alpha-terpinene, cis-ocimene, alpha-thujene, delta-3-carene, gamma-terpinene, alpha-pinene, beta-pinene, limonene, and combinations thereof. Generally, the mint flavor compositions herein contain higher amounts of these C₁₀H₁₆ monoterpenes as compared to commercialized versions assessed. Preferably the composition comprises from 9.2 - 20, preferably 9.5 - 15, more preferably 10.0 - 13, alternatively 9.60 - 11.50, of peak area percent of the C₁₀H₁₆ monoterpenes, as determined by Lei-Hoke Method I. The mint flavor compositions may comprise at least 3, preferably at least 4, more preferably at least 5, yet more preferably at least 6, yet still more preferably at least 7, yet still even more preferably at least 8, alternatively any combination of 1 - 11, of the aforementioned C₁₀H₁₆ monoterpenes. Preferably the C₁₀H₁₆ monoterpenes comprise at least alpha-pinene, beta-pinene, and limonene. More preferably the C₁₀H₁₆ monoterpenes comprise at least alpha-pinene, beta-pinene, limonene, and sabinene. Preferably the C₁₀H₁₆ monoterpenes comprise at least (-)-limonene, preferably from 4.00 - 7, preferably 4.30 - 6 peak area percent of the (-)-limonene, as determined by Lei-Hoke Method V.

In one example, the mint flavor compositions may comprise 6.50 - 15.0, preferably 7.0 - 14, more preferably 7.5 - 12, yet more preferably 8 - 11, peak area percent of an (-)-isomer of the C₁₀H₁₆ monoterpenes, as determined by Lei-Hoke Method V, wherein the (-)-isomer of the C₁₀H₁₆ monoterpenes comprises (-)-alpha-pinene, (-)-beta-pinene, and (-)-limonene. In another example, the mint flavor composition may comprise 1.10 - 1.35, peak area percent of an (+)-isomer of the C₁₀H₁₆ monoterpenes, as determined by Lei-Hoke Method V; and wherein the (+)-isomer of the C₁₀H₁₆ monoterpenes comprises (+)-alpha-pinene, (+)-beta-pinene, and (+)-limonene.

Alpha-pinene is an example of a C₁₀H₁₆ bicyclic monoterpene. The mint flavor composition may comprise from 1.90 - 5.0, preferably 2.00 - 4.0, more preferably 2.2 - 3.5 of peak area percent of alpha-pinene, as determined by Lei-Hoke Method I. The composition may have a peak area ratio of (-)-alpha-pinene : (+)-alpha-pinene from 3.0 - 6, preferably 3.1 - 5, more preferably 3.2 - 4.7, as determined by Lei-Hoke Method IV.

Beta-pinene is an example of a C₁₀H₁₆ bicyclic monoterpene. The mint flavor composition may comprise from 2.2 - 5.0, preferably 2.3 - 4.0, preferably 2.4 - 3.0, peak area percent of beta-pinene, as determined by Lei-Hoke Method I. The composition may have a peak area ratio of (-)-beta-pinene : (+)-beta-pinene from 3 - 8, preferably 4 - 7, more preferably 4.7 - 6.0, as determined by Lei-Hoke Method IV. The composition may comprise from 1.1 - 5, preferably 1.2 - 3, more preferably 1.5 - 2.5, alternatively 2.0 - 2.4, of peak area percent of (-)-beta-pinene, as determined by Lei-Hoke Method V.

Limonene is an example of a C₁₀H₁₆ cyclic monoterpene. The mint flavor composition may comprise from 3.80 - 8, preferably 4.00 - 7, more preferably 4.30 - 6.50, alternatively 4.0 - 6.0 or 4.40 - 5.60, peak area percent, as determined by Lei-Hoke Method I. The composition may have a peak area ratio of (-)-limonene : (+)-limonene from 5 - 40, preferably from 11 - 35, as determined by Lei-Hoke Method IV.

Sabinene is an example of a bicyclic C₁₀H₁₆ monoterpene. The mint flavor composition may comprise 0.1 - 0.4, preferably 0.15 - 0.30, peak area percent of sabinene, as determined by Lei-Hoke Method I.

Without wishing to be bound by theory, the specific terpene amounts and terpene enantiomeric ratios described herein contribute to the success of the flavor profile and its cost effectiveness.

### Use of Fractional Distillates

Certain fractional distillates of the *Mentha* genus plant (e.g., leaves) can be used as inexpensive sources of certain mint flavor components. These fractional distillates described herein are either before or after the typically desired so called "middle-cut" that are used in classic mint oil distillation. It is surprising that these generally undesirable, and thus low cost, distillate fractions can be used for making the mint flavor compositions herein. Preferably these distillates minimize the amount of sulfur-containing compounds that can otherwise impart undesirable flavors, odors, or malodor precursors. In a "front-cut" fractional distillate, are those components having relatively low boiling points, that may include desirable mint flavor components such as limonene, and preferably also pinenes and /or eucalyptol. In a late fractional distillate or "tail-cut", i.e., those components with relatively high boiling points, desirable mint flavor components may include viridiflorol and optionally, but preferably also, germacrene D.

An aspect of the invention provides for a method of making a flavor / mint flavor composition comprising the steps: (a) steam distilling *Mentha* genus plant matter to produce a first mint distillate, wherein the first mint distillate comprises at least 25 peak area percent of limonene, as determined by Lei-Hoke Method I; wherein the first mint distillate further comprises at least 25 peak area percent of one or more mint flavor components, as determined by Lei-Hoke Method I, wherein each of these mint flavor components have a boiling point from 155 - 183 degrees Celsius (and exclusive of limonene); and (b) combining the produced first mint distillate to an additional mint flavor component such that the first mint distillate comprises 0.5% - 6.0% by weight of the flavor / mint flavor composition. The table of Figure 11 describes the mint flavor components, and peak area percent thereof, of a non-limiting example of a first mint distillate. One commercial example of a first mint distillate is the "Mint Oil Terpenes ("front cut")" described in Tables C(1) and C(2) below. Preferably the first mint distillate comprises from 25 - 75, preferably 30 - 70, more preferably 35 - 65, yet more preferably 40 - 60, alternatively 45 - 55, peak area percent of limonene, as determined by Lei-Hoke Method I. Preferably those mint flavor components having a boiling point of 155 - 183 degrees Celsius (excluding limonene) are selected from: alpha-pinene, camphene, sabinene, beta-pinene, myrcene, alpha-terpinene, 3-octanol, eucalyptol, p-cymene, cis-ocimene, gamma-terpinene, and combinations thereof. Preferably the first mint distillate comprises 25 - 75, preferably 30 - 70, more preferably 35 - 65, yet more preferably 40 - 60, alternatively 50 - 55, peak area percent of the mint flavor components having the boiling point from 155 - 183 degrees Celsius (excluding limonene).

Pinenes, e.g., alpha-pinene and beta-pinene, are a specific example of such mint flavor components. Preferably the first mint distillate comprises a pinene, preferably at least 15, preferably at least 20, more preferably 22 - 40, yet more preferably 25 - 35, peak area percent of pinene, per Lei-Hoke Method I. Preferably the pinene is beta-pinene and/or alpha-pinene. In one example, the pinene is a beta-pinene, wherein the first mint distillate comprises at least 5, more preferably at least 10, even more preferably 10 - 25, yet even more preferably 12 - 20, peak area percent of beta-pinene, per Lei-Hoke Method I. In another example, the pinene is an alpha-pinene, wherein the first mint distillate comprises at least 5, more preferably at least 7, yet more preferably 8 - 20, yet still more preferably 10 - 15, peak area percent of alpha-pinene, per Lei-Hoke Method I. In yet another example, the pinene comprises both the alpha-pinene and the beta-pinene, preferably at the aforementioned peak area percent levels.

Eucalyptol is another specific example of such mint flavor components. Preferably the first mint distillate further comprises eucalyptol, preferably at least 1, more preferably at least 3, yet more preferably 3 - 10, yet still more preferably 4 - 8 peak area percent of eucalyptol in the first mint distillate, per Lei-Hoke Method I. Sabinene is another example of such mint flavor components. Preferably the first mint distillate further comprises sabinene, preferably at least 1, more preferably 2 - 6 peak area percent of sabinene, as determined by Lei-Hoke Method I. para-Cymene is another example of such mint flavor components. Preferably the first mint distillate further comprises para-cymene, preferably at least 1, more preferably 2 - 8 peak area percent of para-cymene, as determined by Lei-Hoke Method I.

Preferably, the first mint fractional distillate further comprising mint flavor components selected from the group consisting of camphene, myrcene, alpha-terpinene, 3-octanol, cis-ocimene, gamma-terpinene, and combinations thereof. More preferably, the first mint distillate further comprises at least 2, preferably at least 3, more preferably at least 4, yet more preferably at least 5, yet still more preferably 6, of the aforementioned mint flavor components. In another example, the first fractional distillate comprises from 0.1- 2, preferably 0.5 - 1.5, more preferably 0.8 - 1.2, peak area percent of camphene, as determined by Lei-Hoke Method I. In another example, the first fractional distillate comprises 12- 22, preferably 14 - 20, more preferably 15 - 19, yet more preferably from 16 - 18, peak area percent of beta-pinene, as determined by Lei-Hoke Method I. In another example, the first fractional distillate comprises from 0.5 - 5, preferably 1 - 4, more preferably 2-3, peak area percent of myrcene, as determined by Lei-Hoke Method I. In another example, the first fractional distillate comprises from 0.1 - 2, preferably 0.5 - 1.5, more preferably 0.7 - 1.1, peak area percent of alpha-terpinene, as determined by Lei-Hoke Method I. In another example, the first fractional distillate comprises from 0.5 - 6, preferably 1-5, more preferably 2-4 peak area percent of 3-octanol, as determined by Lei-Hoke Method I. In another example, the first fractional distillate comprises from 25 - 75, preferably 30 - 70, more preferably 35 - 65, yet more preferably 40 - 60, alternatively 42 - 52, peak area percent of limonene, as determined by Lei-Hoke Method I. In another example, the first fractional distillate comprises from 1-11, preferably 2 - 10, more preferably 3 - 9, yet more preferably 4-8, peak area percent of eucalyptol, as determined by Lei-Hoke Method I. In another example, the first fractional distillate comprises from 0.1 - 1, preferably 0.15 - 0.9, more preferably 0.2 - 0.7, peak area percent of cis-ocimene, as determined by Lei-Hoke Method I. In another example, the first fractional distillate comprises from 0.1 - 3, preferably 0.2 - 2, more preferably 0.5 - 1.5, peak area percent of gamma-terpinene, as determined by Lei-Hoke Method I.

Preferably the first mint distillate comprises less than 1,000 part per million (PPM - weight/weight (wt/wt)), preferably less than 200 PPM, more preferably less than 30 PPM of a sulfur compound. Preferably the sulfur compound is selected from dimethyl sulfide, dimethyl sulfoxide, dimethyl disulfide, dimethyl trisulfide, and combinations thereof. Preferably the sulfur compound is dimethyl sulfide. The first mint distillate may comprise less than 5, preferably less than 3, more preferably less than 1 peak area percent of additional flavor components that are menthone and menthol, as determined by Lei-Hoke Method I. Preferably the first mint distillate contains less than 1 wt% of C₁-C₃ alcohol, preferably is substantially free of C₁-C₃ alcohol (e.g., ethanol or menthol).

The method of making the flavors / mint flavor compositions may comprise the additional step of combining a second ("tail-cut") mint distillate to the first mint distillate and additional mint flavor component. Preferably the second mint distillate comprises 0.01 - 5.0 percent by weight of the final flavor / mint flavor composition. One commercial example of a second mint distillate is the "Peppermint Residue Distillate ("tail cut")" described in Tables C(1) and C(2) below. The second mint distillate comprises: (i) at least 10%, preferably at least 15%, more preferably at least 20%, yet more preferably at least 25%, of viridiflorol by weight of the second mint distillate; and (ii) less than 30%, preferably less than 20%, more preferably less than 15%, yet more preferably less than 10%, of mintsulfide by weight of the second mint distillate. The second mint distillate optionally comprises, but preferably, germacrene D. If present, the second mint distillate comprises at least 0.1%, more preferably at least 0.5%, yet more preferably 1 - 10% of the germacrene D, by weight of the second mint distillate. Preferably the second mint distillate contains less than 1 wt% of C₁-C₃ alcohol, preferably is substantially free of C₁-C₃ alcohol (e.g., ethanol or menthol).

The first and optionally second mint distillates can be combined with one or more additional mint flavor component(s) as previously described, in the method of making mint flavor compositions and/or flavors containing the same. The method of any one of the preceding claims, wherein the step of adding additional mint flavor components comprising adding synthetic additional mint flavor components such that the flavor / mint flavor composition comprises greater than 80%, preferably greater than 85%, more preferably greater than 90%, even more preferably greater than 93%, synthetic mint flavor components by weight of the flavor / mint flavor composition.

### Additional Mint Flavor Components

In addition to the mint flavor components described herein above, the mint flavor compositions may comprise an additional mint flavor component. The additional mint flavor component is selected from the group consisting of menthone, isomenthone, alpha-pinene, beta-pinene, limonene, menthol, neomenthol, isomenthol, neoisomenthol, menthyl acetate, linalool, terpinen-4-ol, isopulegol, piperitone, dihydromint lactone, eucalyptol, thymol, viridiflorol, 3-hexen-1-ol, menthofuran, caryophyllene, carvone, sabinene, myrcene, camphene, alpha-terpinene, cis-ocimene, alpha-thujene, delta-3-carene, gamma-terpinene, 3-octanol, trans-sabinene hydrate, germacrene D, delta-cadinene, p-cymene, pulegone, alpha-terpineol, and combinations thereof. Preferably the mint flavor composition comprises any one or more combination of 1-37 of the aforementioned additional mint flavor components. More preferably, the mint flavor composition comprises at least 10, more preferably at least 15, yet more preferably at least 20, yet still more preferably at least 25, yet still even more preferably at least 30 of the aforementioned additional mint flavor components. Even more preferably, the mint flavor composition comprises any one or more of the following additional mint flavor components:
(a) 3-hexen-1-ol; preferably from 0.01 - 0.1, more preferably 0.01 - 0.05, yet more preferably 0.01 - 0.03 of peak area percent of 3-hexen-1-ol, as determined by Lei-Hoke Method I;
(b) from 9.2 - 20, preferably 9.5 - 15, more preferably 10.0 - 13 of peak area percent of C₁₀H₁₆ monoterpenes, as determined by Lei-Hoke Method I; preferably wherein the C₁₀H₁₆ monoterpenes comprise at least 1-5, preferably at least 5, selected from the following: sabinene, myrcene, camphene, alpha-terpinene, cis-ocimene, alpha-thujene, delta-3-carene, gamma-terpinene, alpha-pinene, beta-pinene, and limonene;
(c) less than 3.5, preferably 0.01 - 2.2, more preferably 0.1 - 2, even more preferably 0.2 - 1.8 peak area percent in total content of neomenthol, isomenthol, and/or neoisomenthol, as determined by Lei-Hoke Method I;
(d) menthol; preferably from 40.0 - 45.0, preferably 41.5 - 45.0 peak area percent of the menthol, as determined by Lei-Hoke Method I;
(e) (+)- and (-)-menthol; preferably wherein a peak area ratio of (+)-menthol : (-)-menthol is from 0.2 - 0.4, preferably 0.21 - 0.35, as determined by Lei-Hoke Method II;
(f) menthol and menthone; preferably wherein a peak area ratio of menthol : menthone is from 1.6 - 2, preferably 1.7 - 1.9, as determined by Lei-Hoke Method I;
(g) dihydromint lactone; preferably dihydromint lactone is from 0.035 - 0.500, preferably 0.040 - 0.300, more preferably 0.045 - 0.100 of peak area percent, as determined by Lei-Hoke Method I;
(h) (-)-limonene; preferably 4.00 - 7, preferably 4.30 - 6 of peak area percent, as determined by Lei-Hoke Method V;
(i) alpha-pinene; preferably the alpha-pinene is from 1.90 - 5, preferably 2.00 - 4, more preferably 2.20 - 3.5 of peak area percent, as determined by Lei-Hoke Method I; more preferably a peak area ratio of (-)-alpha-pinene : (+)-alpha-pinene is from 3.0 - 6, preferably 3.1 - 5, preferably 3.2 - 4.7 as determined by Lei-Hoke Method IV;
(j) (-)-beta-pinene; preferably from 1.1 - 5, preferably 1.2 - 3, more preferably 1.5 - 2.5, of peak area percent of the (-)-beta-pinene, as determined by Lei-Hoke Method V;
(k) beta-pinene; preferably wherein the beta-pinene is from 2.2 - 5.0, preferably 2.3 - 4.0, preferably 2.4 - 3.0, peak area percent, as determined by Lei-Hoke Method I; more preferably wherein a peak area ratio of (-)-beta-pinene : (+)-beta-pinene is from 3 - 8, preferably 4 - 7, more preferably 4.7 - 6.0, as determined by Lei-Hoke Method IV;
(l) (-)-linalool; preferably the (-)-linalool is from 0.117 to 0.2, preferably 0.120 - 0.200, more preferably 0.125 - 0.190 of peak area percent, as determined by Lei-Hoke Method V; preferably a peak area ratio of (-)-linalool : (+)-linalool is from 0.5 - 2.5, preferably 0.9 - 2.3, as determined by Lei-Hoke Method IV;
(m) menthyl acetate; preferably the menthyl acetate has a peak area percent from 5.5 - 6.5, preferably 5.8 - 6.5, as determined by Lei-Hoke Method I; more preferably the peak area ratio of (+)-menthyl acetate: (-)-menthyl acetate is from 0.1 - 0.980, preferably 0.7 - 0.980, as determined by Lei-Hoke Method II;
(n) menthyl acetate, eucalyptol, and menthofuran; preferably a peak area ratio of menthyl acetate : menthofuran is from 60 - 225, preferably 61 - 200, more preferably 62 - 185, as determined by Lei-Hoke Method I; more preferably a peak area ratio of eucalyptol : menthofuran is from 40 - 115, as determined by Lei-Hoke Method I;
(o) (+)-neomenthol; preferably at a peak area percent of (+)-neomenthol from 0.2 - 1.5, preferably 0.4 - 1, as determined by Lei-Hoke Method V;
(p) (-)- and (+)-neomenthol; preferably a peak area ratio of (-)-neomenthol : (+)-neomenthol from 0 - 0.95, preferably 0.2 - 0.90, as determined by Lei-Hoke Method II;
(q) menthol and neomenthol; preferably wherein a peak area ratio of menthol : neomenthol is from 19 - 80, preferably 25 - 60, more preferably 30 - 55, as determined by Lei-Hoke Method I;
(r) isomenthone; preferably a peak area percent of isomenthone is from 5 - 10, preferably 5.2 - 9, as determined by Lei-Hoke Method I;
(s) limonene; preferably a peak area percent of limonene is from 3.80 - 8, preferably 4.00 - 7, more preferably 4.30 - 6.50, as determined by Lei-Hoke Method I; more preferably a peak area ratio of (-)-limonene : (+)-limonene, is from 5 - 40, preferably 11- 35, as determined by Lei-Hoke Method IV;
(t) thymol; preferably the peak area percent of thymol is from 0.03 - 0.15, preferably 0.05 - 0.10, as determined by Lei-Hoke Method I;
(u) eucalyptol; preferably eucalyptol is from 3 - 5.5, preferably 3.5 - 5 of a peak area percent, as determined by Lei-Hoke Method I;
(v) menthofuran; preferably menthofuran is from 0.01 - 0.1 of peak area percent, as determined by Lei-Hoke Method I;
(w) piperitone; preferably the piperitone is from 0.1 - 1.0, preferably 0.2 - 0.7 of peak area percent, as determined by Lei-Hoke Method I; more preferably a peak area ratio of (-)-piperitone : (+)-piperitone from 2 - 18, preferably 5 - 15, as determined by Lei-Hoke Method IV; and
(x) isopulegol; preferably the isopulegol is from 0.20 - 0.60, preferably 0.21 - 0.50 of a peak area percent, as determined by Lei-Hoke Method I.

Yet even more preferably, the mint flavor composition comprises at least 2, preferably at least 4, more preferably at least 6, yet more preferably least 8, yet still more preferably at least 10, yet still even more preferably at least 12 of the aforementioned additional flavor components (a) - (x). Alternatively, the mint flavor composition comprises any combination of (a) - (x) of said additional flavor components.

### Flavors

Flavors of the present invention comprise a mint flavor composition (as previously defined) and optional ingredients. These optional ingredients may include a wide variety of natural and synthetic non-mint flavor components, minors, and/or solvents. For example, one skilled in the art will add methyl salicylate to the mint flavor compositions described herein to impart a wintergreen flavor profile to the flavor. Another example is the addition of trans-anethole to provide a flavor of the present invention. Non-limiting examples including adding trans-anethole to the mint flavor compositions such that there is from 0.05 - 6% preferably 0.5 -3%, alternatively from 0.9 - 2%, by weight of trans-anethole with respect to the resulting flavor. trans-Anethole (CAS No. 4180-23-8) is identified by its IUPAC name as 1-methoxy-4-[(E)-prop-1-enyl]benzene. Without wishing to be bound by theory, trans-anethole provides licorice-like sweetness and body, and helps smooth or round out the overall flavor profile.

### Consumer Products

An aspect of the present invention comprises a consumer product comprising the mint flavor compositions described herein (or flavors comprising said mint flavor compositions). These consumer products may include foodstuffs such as confectionary, or personal care items such as oral care (e.g., toothpaste and mouthwash). The levels of the mint flavor composition included in the final consumer product are from 0.01 - 10%, preferably 0.1- 5%, more preferably 0.2 - 3%, by weight of the consumer product. Flavors may be contained at similar levels. The consumer product can be selected from foodstuffs (preferably confectionary such as chewing gum) and personal care products (preferably oral care products such as dentifrice).

The mint flavor compositions herein can be incorporated into a variety of consumer products. One aspect of the invention provides a consumer product comprising a carrier and a mint flavor composition. The carrier(s) are the usual and conventional components of the subject consumer product. Foodstuffs can include mint flavor provided by mint flavor compositions herein. One preferred example of foodstuff includes confectionary. In turn, an example of confectionary is chewing gum. Chewing gum generally consists of a water insoluble gum base, a water-soluble portion, and flavor(s). The water-soluble portion dissipates with a portion of the flavor over a period of time during chewing. The gum base portion is retained in the mouth throughout the chew. The insoluble gum base generally comprises elastomers, resins, fats and oils, softeners, and inorganic fillers. The gum base may or may not include wax. A chewing gum formulation may include: sugar (from about 45 wt% to 60 wt%), gum base (from 15 wt% to 30 wt%), corn syrup (from 5 wt% to 10 wt%,), dextrose (from 5 wt% to 20 wt%), glycerin (from 0.1% to 3 wt%), and mint flavor composition as herein described (from 0.1 wt% to 3 wt%, preferably from 0.5 wt% to 2 wt%). Examples of chewing gum are described in US 5,372,824.

Personal care products can include mint flavor provided by mint flavor compositions herein. An oral care product may comprise an aforementioned mint flavor composition and an orally acceptable carrier. Such orally acceptable carriers are materials that include one or more compatible solid or liquid excipients or diluents which are suitable for topical oral administration. By "compatible" it is meant that the components of the composition are capable of being commingled without interaction in a manner which would substantially reduce composition stability, safety, consumer acceptance, and/or efficacy. The carriers can include the usual and conventional components of dentifrices, non-abrasive gels, subgingival gels, mouthwashes or rinses, mouth sprays, chewing gums, lozenges and breath mints as more fully described hereinafter. The choice of a carrier to be used is basically determined by the way the composition is to be introduced into the oral cavity. For example, carrier materials for toothpaste, tooth gel or the like include abrasive materials, sudsing agents, binders, humectants, flavoring and sweetening agents, etc.

In one example, the compositions are in the form of dentifrices, such as toothpastes, tooth gels, tooth powders and tablets. Components of such toothpaste and tooth gels generally include one or more of a dental abrasive (from 6 wt% to 50 wt%), a surfactant (from 0.5 wt% to 10 wt%), a thickening agent (from 0.1 wt% to 5 wt%), a humectant (from 5 wt% to 55 wt%), a flavoring agent (from 0.04 wt% to 3 wt%), a sweetening agent (from 0.1 wt% to 3 wt%), a coloring agent (from 0.01 wt% to 0.5 wt%) and water (from 2 wt% to 45 wt%). Such toothpaste or tooth gel may also include one or more of an anti-caries agent (from 0.05 wt% to 0.3 wt% as fluoride ion) and an anti-calculus agent (from 0.1 wt% to 15 wt%).

In other examples, the compositions are in the form of liquid products, including mouthwashes or rinses, mouth sprays, dental solutions and irrigation fluids. Components of such mouthwashes and mouth sprays typically include one or more of water (from 45 wt% to 95 wt%), ethanol (from 0 wt% to 25 wt%), a humectant (from 0 wt% to 50 wt%), a surfactant (from 0.01 wt% to 7 wt%), a flavoring agent (from 0.04 wt% to 2 wt%), a sweetening agent (from 0.1 wt% to 3 wt%), and a coloring agent (from 0.001 wt% to 0.5 wt%). Such mouthwashes and mouth sprays may also include one or more of an anti-caries agent (from 0.05 wt% to 0.3 wt% as fluoride ion) and an anti-calculus agent (from 0.1 wt% to 3 wt%). Components of dental solutions generally include one or more of water (from 90 wt% to 99 wt%), preservative (from 0.01 wt% to 0.5 wt%), thickening agent (from 0 wt% to 5 wt%), flavoring agent (from 0.04 wt% to 2 wt%), sweetening agent (from 0.1 wt% to 3 wt%), and surfactant (from 0 wt% to 5 wt%). Personal care compositions are described in US 2012/0014883 A1.

### EXAMPLES

### Inventive Flavors comprising Mint Flavor Compositions

**Table C(1) describes the raw materials and compositional ranges for making inventive comprising mint flavor compositions (e.g., inventive examples 1 and 2 of the tables of Figures 1 - 10).**

| MATERIALS | CAS N. | WT/WT % |
|---|---|---|
| (-)-Menthol, Menthol Racemic | 2216-51-5, 89-78-1 | 35-45% |
| Menthone/Isomenthone Racemic | 10458-14-7, 89-80-5, 491-07-6 | 22-26% |
| Menthyl Acetate Racemic | 89-48-5 | 1-12% |
| Synthetic Dementholized Oil (DMO) | Supplier's Proprietary Mixture | 0-20% |
| Eucalyptol | 470-82-6 | 2.3-6.0% |
| Peppermint Residue Distillate ("tail cut") | Mixture | 0.01-5.0% |
| Dihydromint Lactone | 92015-65-1 | 0.035-0.500% |
| L-Limonene | 5989-54-8 | 2.40-8.00% |
| Mint Oil Terpenes ("front cut") | 68608-35-5 | 0.5-6.0% |
| Thymol | 89-83-8 | 0.03-0.15% |
| trans-Anethole | 4180-23-8 | 0.0-3.00% |
| Peppermint Oil | 8006-90-4 | 0-5% |
| Linalool | 78-70-6 | 0.12-0.40% |
| alpha-Pinene | 80-56-8 | 1.0-5.0% |
| beta-Pinene | 127-91-3 | 0.5-3.0% |

**Table C(2) describes the raw materials and compositional ranges for making additional inventive comprising mint flavor compositions (e.g., inventive examples 3 and 4 of the tables of Figures 1 - 10).**

| MATERIALS | CAS No. | WT/WT % |
|---|---|---|
| (-)-Menthol, Menthol Racemic | 2216-51-5, 89-78-1 | 35-45% |
| Menthone/Isomenthone Racemic | 10458-14-7, 89-80-5, 491-07-6 | 22-26% |
| Menthyl Acetate Racemic | 89-48-5 | 1-12% |
| Natural and synthetic mint oil mixture | Supplier's Proprietary Mixture | 0-20% |
| Natural Dementholized Oil | 90063-97-1 | 0-20% |
| Eucalyptol | 470-82-6 | 2.3-6.0% |
| Peppermint Residue Distillate ("tail cut") | Mixture | 0.01-5.0% |
| Dihydromint Lactone | 92015-65-1 | 0.035-0.500% |
| L-Limonene | 5989-54-8 | 2.40-8.00% |
| Mint Oil Terpenes ("front cut") | 68608-35-5 | 0.5-6.0% |
| Thymol | 89-83-8 | 0.03-0.15% |
| trans-Anethole | 4180-23-8 | 0.0-3.00% |
| Peppermint Oil | 8006-90-4 | 0-5% |
| Linalool | 78-70-6 | 0.12-0.40% |
| alpha-Pinene | 80-56-8 | 1.0-5.0% |
| beta-Pinene | 127-91-3 | 0.5-3.0% |

The materials from Tables C(1) and C(2) can be obtained from, but not limited to, the following suppliers: Symrise, BASF, AM Todd, Firmenich, Norwest Ingredients, Bordas, Kerry, H. Reynaud, Takasago, Callisons, Labbeemint, Givaudan, Mane, Sharp Mint Ltd., Copeland, RC Treatt, Penta, Vigon, Sigma Aldrich, Berje, IFF, Excellentia, Global Essence, Robertet, and Lebermuth.

Inventive examples 1 and 2 are the most preferred of the inventive examples because of the even higher cost savings provided compared to inventive examples 3 and 4. As discussed below, inventive examples 2, 3, and 4 tested about equally in their positive mint flavor profile attributes. Inventive example 1 is a slight modification of inventive example 2, and accordingly, is not expected to be significantly different when evaluated among flavor/sensory experts or panelists or consumers.

### Sensory/Flavor Data

Comparative examples A, B, and C are early prototypes that do not display favorably in aroma. Their profiles are thin and lacking robustness. These comparatives are missing a heavy creaminess and/or "smoothing" component to help bring substantivity and fullness to the overall flavor character profile. These comparatives are also too sweet and too clean, lacking in the dank, earthy notes characteristic of natural, hearty mint. To arrive at the current inventions, over 100 iterations were prototyped and evaluated for aroma. During the course of research, as the aroma profile improved to a desired character, mint flavor composition candidates were spiked into toothpaste for quick evaluation by flavor experts. In some cases, mint flavor compositions had favorable aroma profiles, but did not display as well once in the context of finished product. The flavor profile fell flat and was not robust enough to carry the mint impact desired. This undesirably allowed secondary notes of the toothpaste flavor, such as vanilla, spice, or fruity notes, to show through more than the commercial control. Mint flavor compositions that display favorably in both aroma profile and taste in toothpaste are progressed to expert Sensory and Flavor testing described herein with corresponding data shared in Tables E(1), E(2), and F.

Inventive examples 2, 3, and 4 of the mint flavor compositions herein in various dentifrice formulations are compared to that of commercialized versions of the same containing a commercially available mint flavor composition. In Tables E(1) and E(2) below, an initial round of testing is completed by external trained sensory panelists who evaluated and compared the inventive and control dentifrices using a Degree of Difference ("DOD") grading scale. The five-point DOD scale is provided in Table D:

**Table D. Description of Five-point DOD scale:**

| DOD | Definition |
|---|---|
| 1 | No Difference |
| 2 | Slight Difference (may not be able to describe) |
| 3 | Moderate Difference (must be described) |
| 4 | Large Difference (must be described) |
| 5 | Extreme Difference (must be described) |

In Tables E(1) and E(2) below is a summary of the Sensory DOD results from inventive examples 2, 3, and 4 within two Crest^{®} brand dentifrice chassis that are all compared to a commercialized version of the same, with each control also being evaluated against a like version of its respective control.

**Table E (1). Expert panelists evaluation of inventive and comparative dentifrices in tooth whitening dentifrice.**

| Sensory Attribute: | Whitening Dentifrice DOD | | | |
|---|---|---|---|---|
| | Control; Commercialized | Inventive Examples | | |
| | | 2 | 3 | 4 |
| Aroma Difference Rating | 1.71 | 1.83 | 1.8 | 1.62 |
| In Mouth Flavor Difference Rating | 1.57 | 2 | 2 | 1.75 |

**Table E (2). Expert panelists evaluation of inventive and comparative dentifrices in stannous containing dentifrice.**

| Sensory Attribute: | Stannous Containing Dentifrice DOD | | | |
|---|---|---|---|---|
| | Control; Commercialized | Inventive Examples | | |
| | | 2 | 3 | 4 |
| Aroma Difference Rating | 1.14 | 1.33 | 1.6 | 1.86 |
| In Mouth Flavor Difference Rating | 1.57 | 1.5 | 1.8 | 1.86 |

Referencing Tables E(1) and E(2), the data suggests that only slight differences exist between the tested inventive examples (2, 3, and 4) and their respective currently marketed controls. The "negative control legs" (control compared to control) indicate that the sensory panel is performing well in the evaluations. Considering the DOD score for in-mouth evaluations of control vs control was 1.57 for both dentifrice chassis, there is only a maximum 0.5-point difference in scoring for the inventive examples. This data shows the inventive examples 2, 3, and 4 are equivalent in performance to the control.

Due to further cost savings and higher % synthetic composition in inventive Example 2, this inventive mint flavor composition was tested further in nine different dentifrice chassis within multiple flavors. In Table F below, is a summary of the results generated from over 100 specific data points obtained from several time intervals during the brushing experience using nine different dentifrice chassis containing inventive example 2 or the corresponding commercialized version of the same dentifrice (employing a commercialized version of mint flavor).

The data are generated from both an external expert sensory panel and an internal expert flavor panel. Testing is completed by the expert sensory panel by brushing their teeth with both the inventive dentifrice and the respective commercialized version of the same as a control (double blinded, randomized order) with a minimum of one-hour washout period in between using the two dentifrices. The external expert sensory panelists evaluated and provided descriptive feedback comparing inventive with corresponding control dentifrice samples with their conclusions captured in Table F. Internal expert flavor panelists also evaluated the inventive and control dentifrices. These flavorists used the DOD method comparing the inventive dentifrice versus the control by brushing their teeth with both back-to-back (double blinded, randomized order). DOD values are reported as an average on Table F. The five-point DOD scale, for the in-mouth evaluations, is provided previously in Table D herein.

Table F is a comparison by expert sensory and flavor panelists of inventive dentifrice formulations containing inventive example 2 herein, to that of commercialized versions (under the CREST^{®} brand) of the same.

**Table F. Expert panelists evaluation of inventive and comparative dentifrices.**

| Ex. | Dentifrice Chassis | Inventive Ex. 2 (wt%) | Expert Sensory Conclusion / Comments | Expert Flavor DOD & Comments |
|---|---|---|---|---|
| 1 | Baking Soda Peroxide | 0.620% | Very slight differences in cooling observed. Likely not consumer noticeable. | DOD = 1.6; Slightly stronger green herbal notes. |
| 2 | Complete Protection | 0.643% | Minimal differences observed. Interchangeable with current mint. | DOD = 1.5; Very similar |
| 3 | Complete Protection + Whitening | 0.685% | Slight differences in foaming observed. Interchangeable from a flavor perspective. | DOD = 1.6; Very similar |
| 4 | Complete Deep Cleaning | 0.497% | Very similar and interchangeable. | DOD = 2.3; Slightly more musty, spicy character |
| 5 | ProHealth (stannous fluoride) | 0.317% | Very similar and interchangeable. | DOD = 1.5; Very similar |
| 6 | ProHealth + Whitening (stannous fluoride) | 0.406% | Some character differences observed (vanilla and herbal musty). Likely not consumer noticeable. | DOD = 2; Slightly less confectionary character |
| 7 | Whitening | 0.555% | Very similar and interchangeable. | DOD = 2.0; Slightly less sweet, more mentholic |
| 8 | Whitening | 0.295% | Slight character difference observed in aroma (spearmint note). Likely not consumer noticeable. | DOD = 1.8; Similar |
| 9 | Premium Whitening | 0.610% | Very similar and interchangeable. | DOD = 1.5; Slightly harsher mint |

In conclusion, referencing Table F, both expert sensory and expert flavor evaluations show that the differences in mint flavor composition example 2 versus commercialized mint flavor in the context of finished dentifrice product are likely not consumer noticeable and can be interchangeable. Typically, there is risk of consumer noticeable differences around a DOD of 3 (moderate difference), but none of the product pairs were above a DOD of 2.3. Of course, inventive example 2 provides a significant cost savings.

Inventive mint flavor composition of example 1 is a slight modification of example 2 as indicated on the tables of Figures 1-10. These differences are not expected to be significantly sensorially noticeable when evaluated among Flavor/Sensory experts or panelists or consumers; as such, further sensory testing is not warranted.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A mint flavor composition comprising:
(a) a mint flavor component that is dihydromint lactone, wherein the dihydromint lactone is from 0.035 - 0.500, preferably 0.040 - 0.300, more preferably 0.045 - 0.100 of peak area percent, as determined by Lei-Hoke Method I and wherein the composition comprises from about 0.025% to about 0.750% by weight of mint flavor composition of racemic dihydromint lactone, (-)-dihydromint lactone, (+)-dihydromint lactone, and/or combinations thereof; and
(b) an additional mint flavor component, wherein the additional mint flavor component is selected from menthone, isomenthone, alpha-pinene, beta-pinene, limonene, menthol, neomenthol, isomenthol, neoisomenthol, menthyl acetate, linalool, terpinen-4-ol, isopulegol, piperitone, eucalyptol, thymol, viridiflorol, 3-hexen-1-ol, menthofuran, caryophyllene, carvone, sabinene, myrcene, camphene, alpha-terpinene, cis-ocimene, alpha-thujene, delta-3-carene, gamma-terpinene, 3-octanol, trans-sabinene hydrate, germacrene D, delta-cadinene, p-cymene, pulegone, and alpha-terpineol, or combinations thereof.

2. The composition of claim 1 comprising from about 0.035% to about 0.500%, by weight of the mint flavor composition of racemic dihydromint lactone, (-)-dihydromint lactone, (+)-dihydromint lactone, and/or combinations thereof.

3. The composition of any one of the preceding claims, wherein the additional mint flavor component comprises sabinene, myrcene, camphene, alpha-terpinene, cis-ocimene, alpha-thujene, delta-3-carene, gamma-terpinene, or combinations thereof..

4. The composition of any one of the preceding claims, wherein the additional mint flavor component comprises menthol, wherein the menthol has a peak area percent of 40.0 - 45.0, preferably 41.5 - 45.0, as determined by Lei-Hoke Method 1, more preferred wherein the additional mint flavor component comprises (+)- and (-)-menthol, wherein the (+)-menthol : (-)-menthol has a peak area ratio from 0.2 - 0.4, preferably 0.21 - 0.35, as determined by Lei-Hoke Method II.

5. The composition of any one of the preceding claims, wherein the additional mint flavor component comprises menthyl acetate, wherein the menthyl acetate has a peak area percent from 5.5 - 6.5, preferably 5.8 - 6.5, as determined by Lei-Hoke Method I, preferably wherein the additional mint flavor component comprises (+)- and (-)-menthyl acetate, wherein a peak area ratio of (+)-menthyl acetate : (-)-menthyl acetate is from 0.1 - 0.980, preferably 0.7 - 0.980, as determined by Lei-Hoke Method II.

6. The composition of any one of the preceding claims, wherein the additional mint flavor component comprises (+)- and (-)-menthone, wherein the (+)- and (-)-menthone has a peak area percent of 21 - 26, preferably 22.0 - 26.0, as determined by Lei-Hoke Method I.

7. The composition of any one of the preceding claims, wherein the additional mint flavor component comprises (+)- and (-)-menthone, wherein a peak area ratio of (+)-menthone : (-)-menthone is from 0.9 - 1, preferably 0.91 - 0.99, as determined by Lei-Hoke Method III.

8. The composition of any one of the preceding claims, wherein the additional mint flavor component comprises isomenthone, wherein the isomenthone has a peak area percent from 5 - 10, preferably 5.2 - 9, as determined by Lei-Hoke Method 1, preferably wherein the additional mint flavor component comprises (-)- and (+)-isomenthone, wherein a peak area ratio of (-)-isomenthone : (+)-isomenthone is from 0.850 - 0.999, preferably 0.90 - 0.98, as determined by Lei-Hoke Method III.

9. The composition of any one of the preceding claims, wherein the additional mint flavor component comprises from 9.2 - 20; preferably 9.5 - 15; more preferably 10.0 - 13 of peak area percent of C₁₀H₁₆ monoterpenes, as determined by Lei-Hoke Method 1, wherein the C₁₀H₁₆ monoterpene is selected from the group consisting of sabinene, myrcene, camphene, alpha-terpinene, cis-ocimene, alpha-thujene, delta-3-carene, gamma-terpinene, alpha-pinene, beta-pinene, limonene, and combinations thereof, preferably wherein the C₁₀H₁₆ monoterpenes comprise at least five selected from the following: sabinene, myrcene, camphene, alpha-terpinene, cis-ocimene, alpha-thujene, delta-3-carene, gamma-terpinene, alpha-pinene, beta-pinene, and limonene.

10. The composition of any one of the preceding claims, wherein the additional mint flavor component comprises C₁₀H₁₆ monoterpenes, wherein the C₁₀H₁₆ monoterpenes comprise:
(i) from 6.5 - 15.0, preferably 7.0 - 14, more preferably 7.5 - 12 peak area percent of an (-)-isomer of the C₁₀H₁₆ monoterpenes, as determined by Lei-Hoke Method V, and wherein the (-)-isomer the C₁₀H₁₆ monoterpenes comprise (-)-alpha-pinene, (-)-beta-pinene, and (-)-limonene; and
(ii) from 1.10 - 1.35 peak area percent of an (+)-isomer of the C₁₀H₁₆ monoterpenes, as determined by Lei-Hoke Method V, and wherein the (+)-isomer of the C₁₀H₁₆ monoterpenes comprise (+)-alpha-pinene, (+)-beta-pinene, and (+)-limonene.

11. The composition of any one of the preceding claims, wherein the additional mint flavor component is selected from at least 1, preferably at least 2, more preferably at least 3, or 4-13, of any one of the following:
(a) 3-hexen-1-ol; preferably from 0.01 - 0.1, more preferably 0.01 - 0.05, yet more preferably 0.01 - 0.03 of peak area percent of 3-hexen-1-ol, as determined by Lei-Hoke Method I;
(b) from 9.2 - 20, preferably 9.5 - 15, more preferably 10.0 - 13 of peak area percent of C₁₀H₁₆ monoterpenes, as determined by Lei-Hoke Method I, wherein the C₁₀H₁₆ monoterpene is selected from the group consisting of sabinene, myrcene, camphene, alpha-terpinene, cis-ocimene, alpha-thujene, delta-3-carene, gamma-terpinene, alpha-pinene, beta-pinene, limonene, and combinations thereof; preferably wherein the C₁₀H₁₆ monoterpenes comprise at least five selected from the following: sabinene, myrcene, camphene, alpha-terpinene, cis-ocimene, alpha-thujene, delta-3-carene, gamma-terpinene, alpha-pinene, beta-pinene, and limonene;
(c) less than 3.5, preferably 0.01 - 2.2, more preferably 0.1 - 2, even more preferably 0.2 - 1.8 peak area percent in total content of neomenthol, isomenthol, and/or neoisomenthol, as determined by Lei-Hoke Method I;
(d) menthol; preferably from 40.0 - 45.0, preferably 41.5 - 45.0 peak area percent of menthol, as determined by Lei-Hoke Method I;
(e) (+)- and (-)-menthol; preferably wherein a peak area ratio of (+)-menthol : (-)-menthol is from 0.2 - 0.4, preferably 0.21 - 0.35, as determined by Lei-Hoke Method II;
(f) menthol and menthone; preferably wherein a peak area ratio of menthol : menthone is from 1.6 - 2, preferably 1.7 - 1.9, as determined by Lei-Hoke Method I;
(g) (-)-limonene; is from 4.00 - 7, preferably 4.30 - 6, of peak area percent, as determined by Lei-Hoke Method V;
(h) alpha-pinene; preferably the alpha-pinene is from 1.90 - 5, preferably 2.00 - 4, more preferably 2.20 - 3.5 of peak area percent, as determined by Lei-Hoke Method I; more preferably a peak area ratio of (-)-alpha-pinene : (+)-alpha-pinene is from 3.0 - 6, preferably 3.1 - 5, more preferably 3.2 - 4.7, as determined by Lei-Hoke Method IV;
(i) (-)-beta-pinene; preferably from 1.1 - 5, preferably 1.2 - 3, more preferably 1.5 - 2.5, of peak area percent of the (-)-beta-pinene, as determined by Lei-Hoke Method V;
(j) beta-pinene; preferably wherein the beta-pinene is from 2.2 - 5.0, preferably 2.3 - 4.0, preferably 2.4 - 3.0, peak area percent, as determined by Lei-Hoke Method I; more preferably wherein a peak area ratio of (-)-beta-pinene : (+)-beta-pinene is from 3 - 8, preferably 4-7, more preferably 4.7 - 6.0, as determined by Lei-Hoke Method IV;
(k) (-)-linalool; preferably the (-)-linalool is from 0.117 to 0.2, preferably 0.120 - 0.200, more preferably 0.125 - 0.190, of peak area percent, as determined by Lei-Hoke Method V; preferably a peak area ratio of (-)-linalool : (+)-linalool is from 0.5 - 2.5, preferably 0.9 - 2.3, as determined by Lei-Hoke Method IV;
(l) menthyl acetate; preferably the menthyl acetate has a peak area percent from 5.5 - 6.5, preferably 5.8 - 6.5, as determined by Lei-Hoke Method I; more preferably the peak area ratio of (+)-menthyl acetate : (-)-menthyl acetate is from 0.1 - 0.980, preferably 0.7 - 0.980, as determined by Lei-Hoke Method II; and
(m) menthyl acetate, eucalyptol, and menthofuran; preferably a peak area ratio of menthyl acetate : menthofuran is from 60 - 225, preferably 61 - 200, more preferably 62 - 185, as determined by Lei-Hoke Method I; more preferably a peak area ratio of eucalyptol : menthofuran is from 40 - 115, as determined by Lei-Hoke Method I.

12. The composition of any one of the preceding claims, wherein the additional mint flavor component is selected from at least 1, preferably at least 2, more preferably at least 3, or 4-10, of any one of the following:
(a) (+)-neomenthol; preferably at a peak area percent from 0.2 - 1.5, preferably 0.4 - 1, as determined by Lei-Hoke Method V;
(b) (-)- and (+)-neomenthol at a peak area percent from 0.1 - 2.60, preferably 0.77 - 2, more preferably 0.8 - 1.5, as determined by Lei-Hoke Method I; preferably a peak area ratio of (-)-neomenthol : (+)-neomenthol from 0 - 0.95, preferably 0.2 - 0.90, as determined by Lei-Hoke Method II;
(c) menthol and neomenthol; preferably wherein a peak area ratio of menthol : neomenthol is from 19 - 80, preferably 25 - 60, more preferably 30 - 55, as determined by Lei-Hoke Method I;
(d) isomenthone; preferably a peak area percent of isomenthone is from 5-10, preferably 5.2 - 9, as determined by Lei-Hoke Method I;
(e) limonene; preferably a peak area percent of limonene is from 3.80 - 8, preferably 4.00 - 7, more preferably 4.30 - 6.50, as determined by Lei-Hoke Method I; more preferably a peak area ratio of (-)-limonene : (+)-limonene, is from 5 - 40, preferably 11 - 35, as determined by Lei-Hoke Method IV;
(f) thymol; preferably the peak area percent of thymol is from 0.03 - 0.15, preferably 0.05 - 0.10, as determined by Lei-Hoke Method I;
(g) eucalyptol; preferably eucalyptol is from 3 - 5.5, preferably 3.5 - 5 of a peak area percent, as determined by Lei-Hoke Method I;
(h) menthofuran; preferably menthofuran is from 0.01 - 0.1 of peak area percent, as determined by Lei-Hoke Method I;
(i) piperitone; preferably the piperitone is from 0.1 - 1.0, preferably 0.2 - 0.7 of peak area percent, as determined by Lei-Hoke Method I; more preferably a peak area ratio of (-)-piperitone : (+)-piperitone is from 2 - 18, preferably 5 - 15, as determined by Lei-Hoke Method IV; and
(j) isopulegol; preferably the isopulegol is from 0.20 - 0.60, preferably 0.21 - 0.50 of a peak area percent, as determined by Lei-Hoke Method I.

13. The composition of any one of the preceding claims, wherein the composition is substantially and/or predominantly synthetic, preferably wherein the mint flavor composition comprises greater than 80%, preferably greater than 85%, more preferably greater than 90%, even more preferably greater than 93%, synthetic mint flavor components by weight of the composition,

14. A consumer product comprising a carrier and from 0.01 - 10%, preferably 0.1 - 5%, more preferably 0.2 - 3%, by weight of the consumer product of a mint flavor composition according to any one of claims 1-13, preferably wherein the consumer product is an oral care product, more preferred wherein the consumer product is a toothpaste, more preferred the toothpaste comprises stannous fluoride.

## Patentansprüche

1. Aromatische Minzgeschmacksstoffzusammensetzung, umfassend:
(a) einen aromatischen Minzgeschmacksstoffbestandteil, der Dihydrominzlacton ist, wobei das Dihydrominzlacton 0,035-0,500, vorzugsweise zu 0,040-0,300, mehr bevorzugt 0,045-0,100, Peakflächenprozent ist, wie durch Lei-Hoke-Verfahren I bestimmt, und wobei die Zusammensetzung von zu etwa 0,025 Gew.-% bis etwa 0,750 Gew.-% der aromatischen Minzgeschmacksstoffzusammensetzung racemisches Dihydrominzlacton, (-)-Dihydrominzlacton, (+)-Dihydrominzlacton und/oder Kombinationen davon umfasst; und
(b) einen zusätzlichen aromatischen Minzgeschmacksstoffbestandteil, wobei der zusätzliche aromatische Minzgeschmacksstoffbestandteil aus Menthon, Isomenthon, Alpha-Pinen, Beta-Pinen, Limonen, Menthol, Neomenthol, Isomenthol, Neoisomenthol, Menthylacetat, Linalool, Terpinen-4-ol, Isopulegol, Piperiton, Eucalyptol, Thymol, Viridiflorol, 3-Hexen-1-ol, Menthofuran, Caryophyllen, Carvon, Sabinen, Myrcen, Kamphen, Alpha-Terpinen, cis-Ocimen, Alpha-Thujen, Delta-3-Caren, Gamma-Terpinen, 3-Octanol, trans-Sabinenhydrat, Germacren D, Delta-Cadinen, p-Cymol, Pulegon und Alpha-Terpineol oder Kombinationen davon ausgewählt ist.

2. Zusammensetzung nach Anspruch 1, umfassend von zu etwa 0,035 Gew.-% bis etwa 0,500 Gew.-% der aromatischen Minzgeschmacksstoffzusammensetzung racemisches Dihydrominzlacton, (-)-Dihydrominzlacton, (+)-Dihydrominzlacton und/oder Kombinationen davon.

3. Zusammensetzung nach einem der vorstehenden Ansprüche, wobei der zusätzliche aromatische Minzgeschmacksstoffbestandteil Sabinen, Myrcen, Kamphen, Alpha-Terpinen, cis-Ocimen, Alpha-Thujen, Delta-3-Caren, Gamma-Terpinen oder Kombinationen davon umfasst.

4. Zusammensetzung nach einem der vorstehenden Ansprüche, wobei der zusätzliche aromatische Minzgeschmacksstoffbestandteil Menthol umfasst, wobei das Menthol ein Peakflächenprozent von 40,0-45,0, vorzugsweise 41,5-45,0, aufweist, wie durch Lei-Hoke-Verfahren I bestimmt, mehr bevorzugt wobei der zusätzliche aromatische Minzgeschmacksstoffbestandteil (+)- und (-)-Menthol umfasst, wobei das (+)-Menthol : (-)-Menthol ein Peakflächenverhältnis von 0,2-0,4, vorzugsweise 0,21-0,35, aufweist, wie durch Lei-Hoke-Verfahren II bestimmt.

5. Zusammensetzung nach einem der vorstehenden Ansprüche, wobei der zusätzliche aromatische Minzgeschmacksstoffbestandteil Menthylacetat umfasst, wobei das Menthylacetat ein Peakflächenprozent von 5,5-6,5, vorzugsweise 5,8-6,5, aufweist, wie durch Lei-Hoke-Verfahren I bestimmt, vorzugsweise wobei der zusätzliche aromatische Minzgeschmacksstoffbestandteil (+)- und (-)-Menthylacetat umfasst, wobei ein Peakflächenverhältnis von (+)-Menthylacetat : (-)-Menthylacetat von 0,1-0,980, vorzugsweise 0,7-0,980, ist, wie durch Lei-Hoke-Verfahren II bestimmt.

6. Zusammensetzung nach einem der vorstehenden Ansprüche, wobei der zusätzliche aromatische Minzgeschmacksstoffbestandteil (+)- und (-)-Menthon umfasst, wobei das (+)- und (-)-Menthon ein Peakflächenprozent von 21-26, vorzugsweise 22,0-26,0, aufweist, wie durch Lei-Hoke-Verfahren I bestimmt.

7. Zusammensetzung nach einem der vorstehenden Ansprüche, wobei der zusätzliche aromatische Minzgeschmacksstoffbestandteil (+)- und (-)-Menthon umfasst, wobei ein Peakflächenverhältnis von (+)-Menthon : (-)-Menthon von 0,9-1, vorzugsweise 0,91-0,99, ist, wie durch Lei-Hoke-Verfahren III bestimmt.

8. Zusammensetzung nach einem der vorstehenden Ansprüche, wobei der zusätzliche aromatische Minzgeschmacksstoffbestandteil Isomenthon umfasst, wobei das Isomenthon ein Peakflächenprozent von 5-10, vorzugsweise 5,2-9, aufweist, wie durch Lei-Hoke-Verfahren I bestimmt, vorzugsweise wobei der zusätzliche aromatische Minzgeschmacksstoffbestandteil (-)- und (+)-Isomenthon umfasst, wobei ein Peakflächenverhältnis von (-)-Isomenthon : (+)-Isomenthon von 0,850-0,999, vorzugsweise 0,90-0,98, ist, wie durch Lei-Hoke-Verfahren III bestimmt.

9. Zusammensetzung nach einem der vorstehenden Ansprüche, wobei der zusätzliche aromatische Minzgeschmacksstoffbestandteil zu 9,2-20; vorzugsweise 9,5-15; mehr bevorzugt zu 10,0-13, Peakflächenprozent C₁₀H₁₆-Monoterpene umfasst, wie durch Lei-Hoke-Verfahren I bestimmt, wobei das C₁₀H₁₆-Monoterpen ausgewählt ist aus der Gruppe, bestehend aus Sabinen, Myrcen, Kamphen, Alpha-Terpinen, cis-Ocimen, Alpha-Thujen, Delta-3-Caren, Gamma-Terpinen, Alpha-Pinen, Beta-Pinen, Limonen und Kombinationen davon, vorzugsweise wobei die C₁₀H₁₆-Monoterpene wenigstens fünf ausgewählt aus den Folgenden umfassen: Sabinen, Myrcen, Kamphen, Alpha-Terpinen, cis-Ocimen, Alpha-Thujen, Delta-3-Caren, Gamma-Terpinen, Alpha-Pinen, Beta-Pinen und Limonen.

10. Zusammensetzung nach einem der vorstehenden Ansprüche, wobei der zusätzliche aromatische Minzgeschmacksstoffbestandteil C₁₀H₁₆-Monoterpene umfasst, wobei die C₁₀H₁₆-Monoterpene umfassen:
(i) von zu 6,5-15,0, vorzugsweise 7,0-14, mehr bevorzugt 7,5-12 Peakflächenprozent ein (-)-Isomer der C₁₀H₁₆-Monoterpene, wie durch Lei-Hoke-Verfahren V bestimmt, und wobei das (-)-Isomer der C₁₀H₁₆-Monoterpene (-)-Alpha-Pinen, (-)-Beta-Pinen und (-)-Limonen umfasst; und
(ii) von zu 1,10-1,35 Peakflächenprozent ein (+)-Isomer der C₁₀H₁₆-Monoterpene, wie durch Lei-Hoke-Verfahren V bestimmt, und wobei das (+)-Isomer der C₁₀H₁₆-Monoterpene (+)-Alpha-Pinen, (+)-Beta-Pinen und (+)-Limonen umfasst.

11. Zusammensetzung nach einem der vorstehenden Ansprüche, wobei der zusätzliche aromatische Minzgeschmacksstoffbestandteil aus wenigstens 1, vorzugsweise wenigstens 2, mehr bevorzugt wenigstens 3 oder 4-13, eines beliebigen des Folgenden ausgewählt ist:
(a) 3-Hexen-1-ol; vorzugsweise von zu 0,01-0,1, mehr bevorzugt 0,01-0,05, noch mehr bevorzugt 0,01- 0,03 Peakflächenprozent 3-Hexen-1-ol, wie durch Lei-Hoke-Verfahren I bestimmt;
(b) von zu 9,2-20, vorzugsweise 9,5-15, mehr bevorzugt 10,0-13, Peakflächenprozent C₁₀H₁₆-Monoterpene, wie durch Lei-Hoke-Verfahren I bestimmt, wobei das C₁₀H₁₆-Monoterpen ausgewählt ist aus der Gruppe, bestehend aus Sabinen, Myrcen, Kamphen, Alpha-Terpinen, cis-Ocimen, Alpha-Thujen, Delta-3-Caren, Gamma-Terpinen, Alpha-Pinen, Beta-Pinen, Limonen und Kombinationen davon; vorzugsweise wobei die C₁₀H₁₆-Monoterpene wenigstens fünf ausgewählt aus den Folgenden umfassen: Sabinen, Myrcen, Kamphen, Alpha-Terpinen, cis-Ocimen, Alpha-Thujen, Delta-3-Caren, Gamma-Terpinen, Alpha-Pinen, Beta-Pinen und Limonen;
(c) zu weniger als 3,5, vorzugsweise 0,01-2,2, mehr bevorzugt 0,1-2, noch mehr bevorzugt 0,2-1,8, Peakflächenprozent Gesamtgehalt an Neomenthol, Isomenthol und/oder Neoisomenthol, wie durch Lei-Hoke-Verfahren I bestimmt;
(d) Menthol; vorzugsweise zu 40,0-45,0, vorzugsweise 41,5-45,0, Peakflächenprozent Menthol, wie durch Lei-Hoke-Verfahren I bestimmt;
(e) (+)- und (-)-Menthol; vorzugsweise wobei ein Peakflächenverhältnis von (+)-Menthol : (-)-Menthol von 0,2-0,4, vorzugsweise 0,21-0,35, ist, wie durch Lei-Hoke-Verfahren II bestimmt;
(f) Menthol und Menthon; vorzugsweise wobei ein Peakflächenverhältnis von Menthol: Menthon von 1,6-2, vorzugsweise 1,7-1,9, ist, wie durch Lei-Hoke-Verfahren I bestimmt;
(g) (-)-Limonen; ist von 4,00-7, vorzugsweise 4,30-6, Peakflächenprozent, wie durch Lei-Hoke-Verfahren V bestimmt;
(h) Alpha-Pinen; vorzugsweise ist Alpha-Pinen 1,90-5, vorzugsweise 2,00-4, mehr bevorzugt 2,20-3,5, Peakflächenprozent, wie durch Lei-Hoke-Verfahren I bestimmt; mehr bevorzugt ist ein Peakflächenverhältnis von (-)-Alpha-Pinen : (+)-Alpha-Pinen von 3,0-6, vorzugsweise 3,1-5, mehr bevorzugt 3,2-4,7, wie durch Lei-Hoke-Verfahren IV bestimmt;
(i) (-)-Beta-Pinen; vorzugsweise von zu 1,1-5, vorzugsweise 1,2-3, mehr bevorzugt 1,5-2,5, Peakflächenprozent das (-)-Beta-Pinen, wie durch Lei-Hoke-Verfahren V bestimmt;
(j) Beta-Pinen; vorzugsweise wobei das Beta-Pinen von 2,2-5,0, vorzugsweise 2,3-4,0, vorzugsweise 2,4-3,0, Peakflächenprozent ist, wie durch Lei-Hoke-Verfahren I bestimmt; mehr bevorzugt wobei ein Peakflächenverhältnis von (-)-Beta-Pinen : (+)-Beta-Pinen von 3-8, vorzugsweise 4-7, mehr bevorzugt 4,7-6,0, ist, wie durch Lei-Hoke-Verfahren IV bestimmt;
(k) (-)-Linalool; vorzugsweise ist das (-)-Linalool von 0,117-0,2, vorzugsweise 0,120-0,200, mehr bevorzugt 0,125-0,190, Peakflächenprozent, wie durch Lei-Hoke-Verfahren V bestimmt; vorzugsweise ein Peakflächenverhältnis von (-)-Linalool : (+)-Linalool von 0,5-2,5, vorzugsweise 0,9-2,3, ist, wie durch Lei-Hoke-Verfahren IV bestimmt;
(l) Menthylacetat; vorzugsweise weist das Menthylacetat ein Peakflächenprozent von 5,5-6,5, vorzugsweise 5,8-6,5, auf, wie durch Lei-Hoke-Verfahren I bestimmt; mehr bevorzugt ist das Peakflächenverhältnis von (+)-Menthylacetat : (-)-Menthylacetat von 0,1-0,980, vorzugsweise 0,7-0,980, wie durch Lei-Hoke-Verfahren II bestimmt; und
(m) Menthylacetat, Eucalyptol und Menthofuran; vorzugsweise ist ein Peakflächenverhältnis von Menthylacetat : Menthofuran von 60-225, vorzugsweise 61-200, mehr bevorzugt 62-185, wie durch Lei-Hoke-Verfahren I bestimmt; mehr bevorzugt ist ein Peakflächenverhältnis von Eucalyptol : Menthofuran von 40-115, wie durch Lei-Hoke-Verfahren I bestimmt.

12. Zusammensetzung nach einem der vorstehenden Ansprüche, wobei der zusätzliche aromatische Minzgeschmacksstoffbestandteil aus wenigstens 1, vorzugsweise wenigstens 2, mehr bevorzugt wenigstens 3 oder 4-10, eines beliebigen des Folgenden ausgewählt ist:
(a) (+)-Neomenthol; vorzugsweise bei einem Peakflächenprozent von 0,2-1,5, vorzugsweise 0,4-1, wie durch Lei-Hoke-Verfahren V bestimmt;
(b) (-)- und (+)-Neomenthol bei einem Peakflächenprozent von 0,1-2,60, vorzugsweise 0,77-2, mehr bevorzugt 0,8-1,5, wie durch Lei-Hoke-Verfahren I bestimmt; vorzugsweise ein Peakflächenverhältnis von (-)-Neomenthol : (+)-Neomenthol von 0-0,95, vorzugsweise 0,2-0,90, wie durch Lei-Hoke-Verfahren II bestimmt;
(c) Menthol und Neomenthol; vorzugsweise wobei ein Peakflächenverhältnis von Menthol: Neomenthol von 19-80, vorzugsweise 25-60, mehr bevorzugt 30-55, ist, wie durch Lei-Hoke-Verfahren I bestimmt;
(d) Isomenthon; vorzugsweise ist ein Peakflächenprozent von Isomenthon von 5-10, vorzugsweise 5,2-9, wie durch Lei-Hoke-Verfahren I bestimmt;
(e) Limonen; vorzugsweise ist ein Peakflächenprozent von Limonen von 3,80-8, vorzugsweise 4,00-7, mehr bevorzugt 4,30-6,50, wie durch Lei-Hoke-Verfahren I bestimmt; mehr bevorzugt ein Peakflächenverhältnis von (-)-Limonen : (+)-Limonen ist von 5-40, vorzugsweise 11-35, wie durch Lei-Hoke-Verfahren IV bestimmt;
(f) Thymol; vorzugsweise ist ein Peakflächenprozent von Thymol von 0,03-0,15, vorzugsweise 0,05-0,10, wie durch Lei-Hoke-Verfahren I bestimmt;
(g) Eucalyptol; vorzugsweise ist Eucalyptol von zu 3-5,5, vorzugsweise 3,5-5 ein Peakflächenprozent, wie durch Lei-Hoke-Verfahren I bestimmt;
(h) Menthofuran; vorzugsweise ist Menthofuran von 0,01-0,1 Peakflächenprozent, wie durch Lei-Hoke-Verfahren I bestimmt;
(i) Piperiton; vorzugsweise ist das Piperiton von 0,1-1,0, vorzugsweise 0,2-0,7, Peakflächenprozent, wie durch Lei-Hoke-Verfahren I bestimmt; mehr bevorzugt ein Peakflächenverhältnis von (-)-Piperiton : (+)-Piperiton ist von 2-18, vorzugsweise 5-15, wie durch Lei-Hoke-Verfahren IV bestimmt; und
(j) Isopulegol; vorzugsweise ist das Isopulegol von 0,20-0,60, vorzugsweise 0,21-0,50, Peakflächenprozent, wie durch Lei-Hoke-Verfahren I bestimmt.

13. Zusammensetzung nach einem der vorstehenden Ansprüche, wobei die Zusammensetzung im Wesentlichen und/oder überwiegend synthetisch ist, vorzugsweise wobei die aromatische Minzgeschmacksstoffzusammensetzung zu über 80 Gew.-%, vorzugsweise über 85 Gew.-%, mehr bevorzugt über 90 Gew.-%, noch mehr bevorzugt über 93 Gew.-%, der Zusammensetzung synthetische aromatische Minzgeschmacksstoffbestandteile umfasst.

14. Endprodukt, umfassend einen Träger und von zu 0,01-10 Gew.-%, vorzugsweise 0,1-5 Gew.-%, mehr bevorzugt 0,2-3 Gew.-%, des Endprodukts eine aromatische Minzgeschmacksstoffzusammensetzung nach einem der Ansprüche 1 bis 13, vorzugsweise wobei das Endprodukt ein Mundpflegeprodukt ist, mehr bevorzugt, wobei das Endprodukt eine Zahnpasta ist, mehr bevorzugt, wobei die Zahnpasta Zinnfluorid umfasst.

## Revendications

1. Composition d'arôme de menthe comprenant :
(a) un composant d'arôme de menthe qui est du dihydromint lactone, dans laquelle le dihydromint lactone est compris entre 0,035 et 0,500, de préférence 0,040 et 0,300, plus préférablement 0,045 et 0,100 du pourcentage de surface du pic, comme déterminé par la méthode Lei-Hoke I et dans laquelle la composition comprend d'environ 0,025 % à environ 0,750 % en poids de composition d'arôme de menthe de dihydromint lactone racémique, (-)-dihydromint lactone, (+)-dihydromint lactone, et/ou leurs combinaisons ; et
(b) un composant d'arôme de menthe supplémentaire, dans laquelle le composant d'arôme de menthe supplémentaire est choisi parmi menthone, isomenthone, alpha-pinène, bêta-pinène, limonène, menthol, néomenthol, isomenthol, néoisomenthol, acétate de mentyhle, linalol, terpinen-4-ol, isopulegol, pipéritone, eucalyptol, thymol, viridiflorol, 3-hexèn-1-ol, menthofurane, caryophyllène, carvone, sabinène, myrcène, camphène, alpha-terpinène, cis-ocimène, alpha-thujène, delta-3-carène, gamma-terpinène, 3-octanol, hydrate de trans-sabinène, germacrène D, delta-cadinène, p-cymène, pulegone et alpha-terpinéol, ou leurs combinaisons.

2. Composition selon la revendication 1 comprenant d'environ 0,035 % à environ 0,500 %, en poids de la composition d'arôme de menthe de dihydromint lactone racémique, (-)-dihydromint lactone, (+)-dihydromint lactone, et/ou leurs combinaisons.

3. Composition selon l'une quelconque des revendications précédentes, dans laquelle le composant d'arôme de menthe supplémentaire comprend du sabinène, myrcène, camphène, alpha-terpinène, cis-ocimène, alpha-thujène, delta-3-carène, gamma-terpinène, ou leurs combinaisons.

4. Composition selon l'une quelconque des revendications précédentes, dans laquelle le composant d'arôme de menthe supplémentaire comprend du menthol, dans laquelle le menthol a un pourcentage de surface du pic de 40,0 à 45,0, de préférence 41,5 à 45,0, comme déterminé par la méthode Lei-Hoke I, plus préférablement dans laquelle le composant d'arôme de menthe supplémentaire comprend (+)- et (-)-menthol, dans laquelle le (+)-menthol : (-)-menthol a un rapport de surface du pic entre 0,2 et 0,4, de préférence 0,21 et 0,35, comme déterminé par la méthode Lei-Hoke II.

5. Composition selon l'une quelconque des revendications précédentes, dans laquelle le composant d'arôme de menthe supplémentaire comprend de l'acétate de menthyle, dans laquelle l'acétate de menthyle a un pourcentage de surface du pic entre 5,5 et 6,5, de préférence 5,8 et 6,5, comme déterminé par la méthode Lei-Hoke I, de préférence dans laquelle le composant d'arôme de menthe supplémentaire comprend (+)- et (-)-acétate de menthyle, dans laquelle un rapport de surface du pic de (+)-acétate de menthyle : (-)-acétate de menthyle est compris entre 0,1 et 0,980, de préférence 0,7 et 0,980, comme déterminé par la méthode Lei-Hoke II.

6. Composition selon l'une quelconque des revendications précédentes, dans laquelle l'arôme de menthe supplémentaire comprend (+)- et (-)-menthone, dans laquelle la (+)- et (-)-menthone a un pourcentage de surface du pic de 21 à 26, de préférence 22,0 à 26,0, comme déterminé par la méthode Lei-Hoke I.

7. Composition selon l'une quelconque des revendications précédentes, dans laquelle le composant d'arôme de menthe supplémentaire comprend (+)- et (-)-menthone, dans laquelle un rapport de surface du pic de (+)-menthone : (-)-menthone est compris entre 0,9 et 1, de préférence 0,91 et 0,99, comme déterminé par la méthode Lei-Hoke III.

8. Composition selon l'une quelconque des revendications précédentes, dans laquelle le composant d'arôme de menthe supplémentaire comprend de l'isomenthone, dans laquelle l'isomenthone a un pourcentage de surface du pic entre 5 et 10, de préférence 5,2 et 9, comme déterminé par la méthode Lei-Hoke I, de préférence dans laquelle le composant d'arôme de menthe supplémentaire comprend (-)- et (+)-isomenthone, dans laquelle un rapport de surface du pic de (-)-isomenthone : (+)-isomenthone est compris entre 0,850 et 0,999, de préférence 0,90 et 0,98, comme déterminé par la méthode Lei-Hoke III.

9. Composition selon l'une quelconque des revendications précédentes, dans laquelle le composant d'arôme de menthe supplémentaire comprend entre 9,2 et 20 ; de préférence 9,5 et 15 ; plus préférablement 10,0 et 13 du pourcentage de surface du pic des monoterpènes en C₁₀H₁₆, comme déterminé par la méthode Lei-Hoke I, dans laquelle le monoterpène en C₁₀H₁₆ est choisi dans le groupe constitué de sabinène, myrcène, camphène ,alpha-terpinène, cis-ocimène, alpha-thujène, delta-3-carène, gamma-terpinène, alpha-pinène, bêta-pinène, limonène et leurs combinaisons, de préférence dans laquelle les monoterpènes en C₁₀H₁₆ comprennent au moins cinq monoterpènes choisis parmi les suivants : sabinène, myrcène, camphène, alpha-terpinène, cis-ocimène, alpha-thujène, delta-3-carène, gamma-terpinène, alpha-pinène, bêta-pinène et limonène.

10. Composition selon l'une quelconque des revendications précédentes, dans laquelle le composant d'arôme de menthe supplémentaire comprend des monoterpènes en C₁₀H₁₆ supplémentaire, dans laquelle les monoterpènes en C₁₀H₁₆ comprennent :
(i) entre 6,5 et 15,0, de préférence 7,0 et 14, plus préférablement 7,5 et 12, pour cent de surface du pic d'un (-)-isomère des monoterpènes en C₁₀H₁₆, comme déterminé par la méthode Lei-Hoke V, et dans laquelle les (-)-isomères des monoterpènes en C₁₀H₁₆ comprennent (-)-alpha-pinène, (-)-béta-pinène et (-)-limonène ; et
(ii) entre 1,10 et 1,35 pour cent de surface du pic d'un (+)-isomère des monoterpènes en C₁₀H₁₆, comme déterminé par la méthode Lei-Hoke v, et dans laquelle le (+)-isomère des monoterpènes en C₁₀H₁₆ comprend (+)-alpha-pinène, (+)-bêta-pinène et le (+)-limonène.

11. Composition selon l'une quelconque des revendications précédentes, dans laquelle le composant d'arôme de menthe supplémentaire est choisi parmi au moins 1, de préférence au moins 2, plus préférablement au moins 3, ou 4 et 13, selon l'un quelconque des éléments suivants :
(a) 3-hexèn-1-ol ; de préférence entre 0,01 et 0,1, plus préférablement 0,01 et 0,05, encore plus préférablement 0,01 et 0,03 du pourcentage de surface du pic de 3-hexène-1-ol, comme déterminé par la méthode Lei-Hoke I ;
(b) entre 9,2 et 20, de préférence 9,5 et 15, plus préférablement 10,0 et 13 du pourcentage de surface du pic des monoterpènes en C₁₀H₁₆, comme déterminé par la méthode Lei-Hoke I, dans laquelle le monoterpène en C₁₀H₁₆ est choisi dans le groupe constitué de sabinène, myrcène, camphène, alpha-terpinène, cis-ocimène, alpha-thujène, delta-3-carène, gamma-terpinène, alpha-pinène, bêta-pinène, limonène et leurs combinaisons ; de préférence dans laquelle les monoterpènes en C₁₀H₁₆ comprennent au moins cinq monoterpènes choisis parmi les suivants : sabinène, myrcène, camphène, alpha-terpinène, cis-ocimène, alpha-thujène, delta-3-carène, gamma-terpinène, alpha-pinène, bêta-pinène et limonène ;
(c) moins de 3,5, de préférence entre 0,01 et 2,2, de préférence entre 0,1 et 2, plus préférablement 0,2 et 1,8 pourcentage de surface du pic en teneur totale de néomenthol, isomenthol et/ou néoisomenthol, comme déterminé par la méthode Lei-Hoke I ;
(d) menthol ; de préférence entre 40,0 et 45,0, de préférence 41,5 et 45,0 pour cent de surface du pic de menthol, comme déterminé par la méthode Lei-Hoke I ;
(e) (+)- et (-)-menthol ; de préférence dans laquelle un rapport de surface du pic de (+)-menthol : (-)-menthol est compris entre 0,2 et 0,4, de préférence 0,21 et 0,35, comme déterminé par la méthode Lei-Hoke II ;
(f) menthol et menthone ; de préférence dans laquelle un rapport de surface du pic de menthol : menthone est compris entre 1,6 et 2, de préférence 1,7 et 1,9, comme déterminé par la méthode Lei-Hoke I ;
(g) (-)-limonène ; est compris entre 4,00 et 7, de préférence 4,30 et 6, du pourcentage de surface du pic, comme déterminé par la méthode Lei-Hoke V ;
(h) alpha-pinène ; de préférence, l'alpha-pinène est compris entre 1,90 et 5, de préférence 2,00 et 4, plus préférablement 2,20 et 3,5 du pourcentage de surface du pic, comme que déterminé par la méthode Lei-Hoke I ; plus préférablement, un rapport de surface du pic de (-)-alpha-pinène : (+)-alpha-pinène est compris entre 3,0 et 6, de préférence 3,1 et 5, plus préférablement 3,2 et 4,7, comme déterminé par la méthode Lei-Hoke IV ;
(i) (-)-bêta-pinène ; de préférence entre 1,1 et 5, de préférence 1,2 et 3, plus préférablement 1,5 et 2,5, du pourcentage de surface du pic du (-)-bêta-pinène, comme déterminé par la méthode Lei-Hoke V ;
(j) bêta-pinène ; de préférence dans laquelle le bêta-pinène est compris entre 2,2 et 5,0, de préférence 2,3 et 4,0, de préférence 2,4 et 3,0, pour cent de surface du pic, comme déterminé par la méthode Lei-Hoke I ; plus préférablement dans laquelle un rapport de surface du pic de (-)-bêta-pinène : (+)-bêta-pinène est compris entre 3 et 8, de préférence 4 et 7, plus préférablement 4,7 et 6,0, comme déterminé par la méthode Lei-Hoke IV ;
(k) (-)-linalol ; de préférence, le (-)-linalool est compris entre 0,117 et 0,2, de préférence 0,120 et 0,200, plus préférablement 0,125 et 0,190, du pourcentage de surface du pic, comme déterminé par la méthode Lei-Hoke V ; de préférence un rapport de surface du pic de (-)-linalol : (+)-linalol est compris entre 0,5 et 2,5, de préférence 0,9 et 2,3, comme déterminé par la méthode Lei-Hoke IV ;
(l) acétate de menthyle ; de préférence, l'acétate de menthyle a un pourcentage de surface du pic compris entre 5,5 et 6,5, de préférence 5,8 et 6,5, comme déterminé par la méthode Lei-Hoke I ; plus préférablement, le rapport de surface du pic de (+)-acétate de menthyle : (-)-acétate de menthyle est compris entre 0,1 et 0,980, de préférence 0,7 et 0,980, comme déterminé par la méthode Lei-Hoke II ; et
(m) acétate de menthyle, eucalyptol et menthofurane ; de préférence un rapport de surface du pic d'acétate de menthyle : menthofurane est compris entre 60 et 225, de préférence 61 et 200, plus préférablement 62 et 185, comme déterminé par la méthode Lei-Hoke I ; plus préférablement un rapport de surface du pic d'eucalyptol : menthofurane est compris entre 40 et 115, comme déterminé par la méthode Lei-Hoke I.

12. Composition selon l'une quelconque des revendications précédentes, dans laquelle le composant d'arôme de menthe supplémentaire est choisi parmi au moins 1, de préférence au moins 2, plus préférablement au moins 3, ou 4 et 10, de l'un quelconque des éléments suivants :
(a) (+)-néomenthol ; de préférence à un pourcentage de surface du pic compris entre 0,2 et 1,5, de préférence 0,4 et 1, comme déterminé par la méthode Lei-Hoke V ;
(b) (-)- et (+)-néomenthol à un pourcentage de surface du pic compris entre 0,1 et 2,60, de préférence 0,77 et 2, plus préférablement 0,8 et 1,5, comme déterminé par la méthode Lei-Hoke I ; de préférence un rapport de surface du pic de (-)-néomenthol : (+)-néomenthol entre 0 et 0,95, de préférence 0,2 et 0,90, comme déterminé par la méthode Lei-Hoke II ;
(c) menthol et néomenthol ; de préférence dans laquelle un rapport de surface du pic de menthol : néomenthol est compris entre 19 et 80, de préférence 25 et 60, plus préférablement 30 et 55, comme déterminé par la méthode Lei-Hoke I ;
(d) isomenthone ; de préférence un pourcentage de surface du pic d'isomenthone est compris entre 5 et 10, de préférence 5,2 et 9, comme déterminé par la méthode Lei-Hoke I ;
(e) limonène ; de préférence un pourcentage de surface du pic de limonène est compris entre 3,80 et 8, de préférence 4,00 et 7, plus préférablement 4,30 et 6,50, comme déterminé par la méthode Lei-Hoke I ; plus préférablement un rapport de surface du pic de (-)-limonène : (+)-limonène, est compris entre 5 et 40, de préférence 11 et 35, comme déterminé par la méthode Lei-Hoke IV ;
(f) thymol ; de préférence, le pourcentage de surface du pic de thymol est compris entre 0,03 et 0,15, de préférence 0,05 et 0,10, comme déterminé par la méthode Lei-Hoke I ;
(g) eucalyptol ; de préférence, l'eucalyptol est compris entre 3 et 5,5, de préférence 3,5 et 5 d'un pourcentage de surface du pic, comme déterminé par la méthode Lei-Hoke I ;
(h) menthofurane ; de préférence, le menthofurane est compris entre 0,01 et 0,1 du pourcentage de surface du pic, comme déterminé par la méthode Lei-Hoke I ;
(i) piperitone ; de préférence, la pipéritone est comprise entre 0,1 et 1,0, de préférence 0,2 et 0,7 du pourcentage de surface du pic, comme déterminé par la méthode Lei-Hoke I ; plus préférablement un rapport de surface du pic de (-)-pipéritone : (+)-pipéritone est compris entre 2 et 18, de préférence 5 et 15, comme déterminé par la méthode Lei-Hoke IV ; et
(j) isopulegol ; de préférence, l'isopulegol est compris entre 0,20 et 0,60, de préférence 0,21 et 0,50 d'un pourcentage de surface du pic, comme déterminé par la méthode Lei-Hoke I.

13. Composition selon l'une quelconque des revendications précédentes, dans laquelle la composition est essentiellement et/ou principalement synthétique, de préférence dans laquelle la composition d'arôme de menthe comprend plus de 80 %, de préférence plus de 85 %, plus préférablement plus de 90 %, encore plus préférablement plus de 93 %, de composants d'arôme de menthe synthétiques en poids de la composition,

14. Produit de consommation comprenant un support et compris entre 0,01 à 10 %, de préférence 0,1 et 5 %, plus préférablement 0,2 et 3 %, en poids du produit de consommation d'une composition d'arôme de menthe selon l'une quelconque des revendications 1 à 13, de préférence dans lequel le produit de consommation est un produit d'hygiène buccale, plus préférablement dans lequel le produit de consommation est un dentifrice, plus préférablement le dentifrice comprend du fluorure d'étain.
